# EUROPEAN PATENT SPECIFICATION

(11) **EP 0 345 906 B1**
(45) Date of publication and mention of the grant of the patent: **13.03.1996**
(21) Application number: 89201460.6
(22) Date of filing: 07.06.1989
(51) Int. Cl.: C07K 7/06, C07K 14/75, A61K 38/36, G01N 33/68

(54) **Unique elastase induced fibrinogen cleavage site antigens**
Antigene der einzigen Spaltungsstelle von Fibrinogen durch Elastase
Antigènes spécifiques du site de coupure unique du fibrinogène par l'élastase

(30) Priority: 10.06.1988 US 205416; 10.06.1988 US 205417; 10.06.1988 US 205418
(43) Date of publication of application: 13.12.1989
(73) Proprietor: MERCK & CO. INC., Rahway New Jersey 07065-0900 (US)
(72) Inventor: Dahlgren, Mary E., Metuchen New Jersey 08840 (US); Mumford, Richard A., Red Bank New Jersey 07701 (US); Boger, Joshua S., Westfield New Jersey 07090 (US); Davies, D.T. Philip, New Jersey 07076 (US)
(74) Representative: Cole, William Gwyn

(56) References cited:
- WO-A-88/08134
- J Lab Clin Med, vol. 102, No. 6 1983, pages 858-869. E F Plow et al.
- XIth International Congress on Thrombosis and Haemostasis, Brussels, 6-10 July 1987, vol.58, no.1, page 301. R Wallin et al.
- XIth International Congress on Thrombosis and Haemostasis, Brussels, 6-10 July 1987, vol. 58, No1, page 104. T Eckhardt et al.
- Proc Natl Acad Sci USA, vol.79 June 1982, pages 3711-3715. EF Plow et al.
- J Clin Invest., vol. 78, July 1986, pages 155-162. The American Society for Clinical Investigation, Inc. J I Weitz et al.

## Description

### BACKGROUND OF THE INVENTION

Human leukocyte proteinases, elastase in particular, have been implicated in the pathogenesis of various connective tissue destructive diseases including pulmonary emphysema, chronic bronchitis, cystic fibrosis, bronchiectasis, acute respiratory distress syndrome, arthritis, glomerulonephritis, psoriasis, vasculitis and atherosclerosis, Janoff, Ann. Rev. Med. 36: 207-216 (1985). Elastase may also be involved in other lung diseases such as PiZZ emphysema and infantile respiratory distress syndrome and in myelogenous leukemia and gout. Elastase, a serine proteinase active at neutral pH, is stored in the azurophilic granules of polymorphonuclear leukocytes and in lysosomes of transient mononuclear leukocytes. Elastase as used herein is defined as elastase derived from leukocytes. Leukocyte as used herein includes neutrophils, macrophages and monocytes. Cellular elastase is released in tissues when the cell encounters matter to be phagocytized or undergoes cellular autolysis, Janoff, supra. Tissue pathology occurs when the released elastase encounters endogenous connective tissue substrates such as elastin, proteoglycan, types III and IV collegan and fibrinonectin, Janoff, Am. Rev. Respir. Dis. 132: 417-433 (1985). In addition to connective tissue components, human leukocyte elastase can hydrolyze various plasma proteins including immunoglobulins, fibrin, fibrinogen and complement proteins.

Extracellular human neutrophil elastase (HLE) activity is controlled by circulating or locally produced elastase inhibitors. Circulating elastase inhibitors include alpha-1-proteinase inhibitor and alpha-2-macroglobulin. Alpha-1-proteinase inhibitor is the most prevalent regulator of endogenous elastase activity and blocks enzyme activity by complexing with the elastase so that it is unable to act on other substrates. The enyzme-inhibitor complexes are then cleared form the tissues, Janoff, Ann. Rev. Med. 36: 207-216 (1985).

Elastase and the other leukocyte associated proteinases have also been implicated in the alternate fibrinolytic pathway responsible for the dissolution of fibrinogen and fibrin, Plow, J. Clin Invest. 69: 564-572 (1982). The fibrinolytic activity may be physiologically important in thrombi reduction but may also lead to pathological conditions related to blood coagulation, Weitz et al., J. Clin. Invest. 78: 155-162 (1986).

A thorough understanding of the role of leukocyte elastase in both physiological and pathological states has been hampered by the absence of a quick, efficient and reliable assay for detecting enzymatic activity in vitro with a natural substrate such as human fibrinogen. Direct measurement of the enzymatic activity of released HLE is difficult because the released enzyme rapidly binds to the substrate or to serum proteinase inhibitors. Bound enzyme may be quickly removed from the circulation, Campbell, et al. Proc. Nat'l Acad. Sci. USA 79: 6941-6955 (1982), Kaplan and Nielsen J. Biol. Chem. 254: 7329-7335 (1979). Immunoassay determinations of released enzyme, Plow, J. Clin. Invest. 69: 564-572 (1982) cannot distinguish between free enzyme and enzyme bound to inhibitor, and determinations of enzyme-inhibitor complexes, Brower and Harpel, Blood 61: 842-849 (1983) have the disadvantage that they detect only the inactivated enzyme.

Since direct detection of enzymatically active HLE in physiological fluids has not proven reliable, alternate assay procedures dependent upon the detection of HLE cleavage products have been designed. Two of these methods have used elastase-cleaved products of elastin as markers. One method measured urinary desmosine, a major cross-linking amino acid of elastin, by radioimmunoassay, King et al., Connect. Tissue Res. 7: 263-267 (1986) and Harel et al., Am. Rev. Resp. Dis. 122: 769-773 (1980). No consistent difference has been observed between healthy individuals and chronic lung disease patients, Pelham et al., Am. Rev. Resp. Dis. 132: 821-823 (1985). A similar result has been reported using ELISA measurements of elastase-generated elastin-derived peptides in serum of lung disease patients and normal controls, Tockman et al., Am. Rev. Resp. Dis. 133: A60 (1986).

Assays designed to detect plasmin-generated fibrinogen degradation products (FDP) have been used to detect fibrinogen cleavage products. Early assays utilized antibodies reactive with human FDP prepared by human plasmin digestion of human fibrinogen at room temperature for 12 to 36 hours, U.S. Patent 3,912,805. The antibodies are attached to red blood cells and are used in passive agglutination assays to detect FDP. Antibodies to purified FDP fragments such as fragments D and E have also been employed to identify and quantify fibrinogen degradation products, U.S. Patent 4,090,846. The assay consisted of an indirect method in which human serum or urine were mixed with the antiserum to the purified D and E fragments, to which was added FDP bound to latex beads. Other fibrinogen fragments have been used to produce antibody that can be used to detect the fragments in blood. Plasminogen derived fragments such as Fg-E have been described by Chen and Schulof, Thrombos. Res. 16: 601-615 (1979) and antibodies to the fragment have been used in a radioimmunoassay to detect the fragment in plasma.

Fibrinogen degradation products prepared by digesting human fibrinogen with HLE have been used to develop as says for evaluating elastase cleavage products in fluids, Plow et al., J. Lab. Clin. Ned. 102: 858-868 (1983). Human fibrinogen was digested with human leukocyte elastase at 1:50 (w/w) enzyme-to-substrate ratio for 24 hrs at 37°C. The predominant fragment was a D-like fragment of 80,000 MW and designated Dₑ. Antibody to this fragment retained its capacity to recognize the Dₑ in radioimmunoassays after adsorption with intact fibrinogen and plasmin degradation products. Plow suggested that this indicated the recognition of an elastase-elicited neoantigen that was only expressed by elastase degradation products. When elastase degradation products were added to normal plasma, the elastase-elicited neoantigen could be quantitatively detected in radioimmunoassay. The principal disadvantage of the fibrinogen degradation product assay procedures is that the material being assayed is defined only operationally. The sites of HLE (or plasmin) cleavage have not been adequately defined. Consequently, the specificity of the antisera cannot be rigorously investigated and the exact nature of the products measured in the immunoassay is unknown.

Immunoassays designed to detect products of thrombin cleavage of fibrinogen have been described in several animal species. For example, a radioimmunoassay has been described which identifies the peptide F-CB1a from the amino end of bovine fibrinogen Aa chain, Tanswell et al. Euro. J. Biochem. 88: 565-571 (1978). Thrombin cleavage of F-CB1 yielded two fragments: fibrinopeptide A (residues 1-19; equivalent to human residues 1-16) and the carboxy-terminal fragment Th2 (residues 20-54; equal to human residues 17-51).

Fibrinopeptide A (FPA), the 1-16 amino acid residue of the Aα chain (Aα 1-16), is released by thrombin cleavage and has been used to produce antibodies which can be employed to assay for the fibrinogen cleavage product FPA, Canfield et al., Biochem. 15: 1203-1209 (1976). Antibodies reactive with FPA were used to study the human elastase cleavage products of human fibrinogen, Bilezikian and Nossel, Blood: 50; 21-28 (1977). It was found that the elastase degradation products were different from the thrombin degradation products as determined by radioimmunoassays specific for FPA. The elastase cleavage product was slightly larger than FPA and could be distinguished from FPA by immunological means.

Eckhardt et al. in Thromb. Haemostasis, 1987, 58(1), 104, No. 366 describe the measurement of elastase-induced fibrinogen-derived peptides in vitro using an assay for measuring peptides produced by the enzymic cleavage. Mention is made of the existence of a peptide Aα 1-21.

Walkin and Saldeen in Thromb. Haemostasis, 1987, 58(1), 301, No. 1100 describe a radioimmunoassay for the measurement of a peptide designated Bβ 40-43, which appears to be produced by the cleavage of fibrinogen by elastase.

Antibody specifically reactive with FPA has been used to evaluate in vivo human leukocyte elastase activity, Weitz et al., J. Clin. Invest. 71: 155-162 (1986). Weitz found that leukocyte elastase cleaves fibrinogen into a fibrinopeptide A-containing fragment which can be used as an index of elastase activity. Human leukocyte elastase cleaves a 21 amino acid peptide, fibrinogen Aα 1-21 form the amino terminus of the alpha chain of human fibrinogen. This Aα 1-21 peptide contains the entire FPA peptide. The Weitz assay does not directly measure the elastase cleavage product, but measures FPA, Aα 1-16, which is the normal human thrombin cleavage product of human fibrinogen, Blomback et al., Nature 275: 501-505 (1978). The Weitz two step radioimmunoassay utilizes fresh human plasma, ethanol treated, presumably totally fibrinogen depleted, either treated or untreated with human thrombin. The difference in immunoreactive FPA, Aα 1-16, between the thrombin treated and untreated samples is attributed to the amount of Aα 1-21 present in the original sample. This assay does not directly measure any elastase cleavage product in body fluids and requires a two-step process to detect what appears to be a single elastase cleavage product.

The present invention allows the detection of one or more specific human elastase cleavage products of human fibrinogen. This is accomplished by the use of specific antibody reactive with the amino or carboxyl termini of the cleavage sites which does not cross react with the intact protein, the products of enzymatic cleavage at other sites, or with other epitopes associated with the cleavage peptides. The assay procedure is a single step assay which allows for the rapid and reproducible detection of specific cleavage peptides. The specific antibodies bind to the cleavage site and do not react with the intact fibrinogen protein.

### OBJECTS THE INVENTION

It is accordingly, an object of the present invention to provide peptide antigens associated with the major human leukocyte elastase (HLE) cleavage products of human fibrinogen. Another object is to synthesize the unique antigens and specifically conjugate them to a high molecular weight carrier protein. A further object is to produce monospecific antibodies against the specific antigens. Another object is to synthesize unique probes to determine antibody specificity and to detect specific antibody. Another object is to provide a simple, rapid and efficient process for the determination of in vivo or in vitro elastase cleavage products. Another object is to provide a kit for the determination of in vivo elastase activity. A further object is to assay for the elastase-inhibitory capacity of a solution, tissue extract or physiological fluid by stimulating the release of HLE from neutrophils or monocytes in a whole blood sample and monitoring the effective HLE activity using the immunoassay kit to measure the production of peptides which are HLE cleavage products. These and other objects of the present invention will be apparent from the following description.

### SUMMARY OF THE INVENTION

Novel peptides associated with the amino and carboxyl termini of human leukocyte elastase cleavage sites of human fibrinogen are disclosed. Antibodies are raised against these peptides which are specific for as few as five amino acids on either side of the enzymatic cleavage site. The peptides and the corresponding antibodies are used in a novel assay for the rapid determination of in vivo elastase cleavage products. The antibodies are specific for the cleavage site peptides without cross-reacting with the intact fibrinogen molecule or with the peptides produced by enzymatic cleavage at other sites. A sample containing elastase-induced fibrinogen cleavage products is contacted with one or more antibodies specific for epitopes associated with peptides of the carboxyl or amino termini of human neutrophil elastase cleaved fibrinogen and incubated. The sample is then contacted with a labeled probe corresponding to the antigen or antigens used to raise the specific antibodies. The amount of antibody-bound probe is determined, and the amount of unlabelled peptide is determined by comparison to a standard curve. Samples of whole blood derived from patients suspected of deficient elastase inhibitory capacity are contacted with an agonist which releases HLE from neutrophils. The elastase activity is assayed by measuring the production of specific HLE cleavage products.

### DETAILED DESCRIPTION OF THE INVENTION

The present invention relates to the identification of time dependent, major human leukocyte elastase cleavage sites within an intact fibrinogen molecule. The structure of mammalian fibrinogen is known in the art and the human fibrinogen amino acid sequence has been schematically depicted by Doolittle, Ann. N.Y. Acad. Sci. 408: 13-26 (1983). The amino acid sequences in human fibrinogen Aα-,Bβ-and γ-chains is also known in the art and have been characterized by Henschen et al., Ann. N.Y. Acad. Sci. 408:28-43 (1983). The enzymatic cleavage sites are determined by reacting human fibrinogen (Sigma), about 200 nano-moles, with HLE (Elastin Products) or monocyte elastase, about 1 nano-mole, at about 37°C for various time periods ranging from about 0 minutes to about 2 hours. The reactions are carried out in an acceptable buffer, preferably Tris, at a pH of about 7.0 in a final volume of about 1.0 ml. Each reaction is stopped by the addition of cold alcohol, preferably ethanol at about 3°C and incubated at about 3°C for about 30 minutes. Following incubation the suspension is centrifuged at about 3,000 X g at about 3°C for about 20 minutes. The supernatant fluid is collected and transferred to clean tubes and concentrated to dryness in a Speed-Vac for about 16 hours. Each sample is reconstituted in an acceptable high performance liquid chromatography (HPLC) buffer, preferably water and acetonitrile mixtures and chromatographed on a C-18 Dupont-ZorbaxT column, about 0.4 x 25 cm. The peptides are eluted with a linear gradient consisting of about H₂O:aceto-nitrile:trifluoroacetic acid 95:5:0.2 v/v/v, to about H₂O:acetonitrile: trifluoroacetic acid 60:40:0.2 v/v/v/. Peptide peaks are collected, re-chromatographed to demonstrate purity and subjected to N-terminal amino acid sequence analysis on an Applied Biosystems Protein Peptide Sequencer, Model 470A. Eight major peaks are identified and the amino acid sequences are used to determine the sites of HLE cleavage. Nineteen minor amino acid peaks are also identified and can be used to identify minor or secondary HLE cleavage sites. The major HLE cleavage sites are shown in the following table.

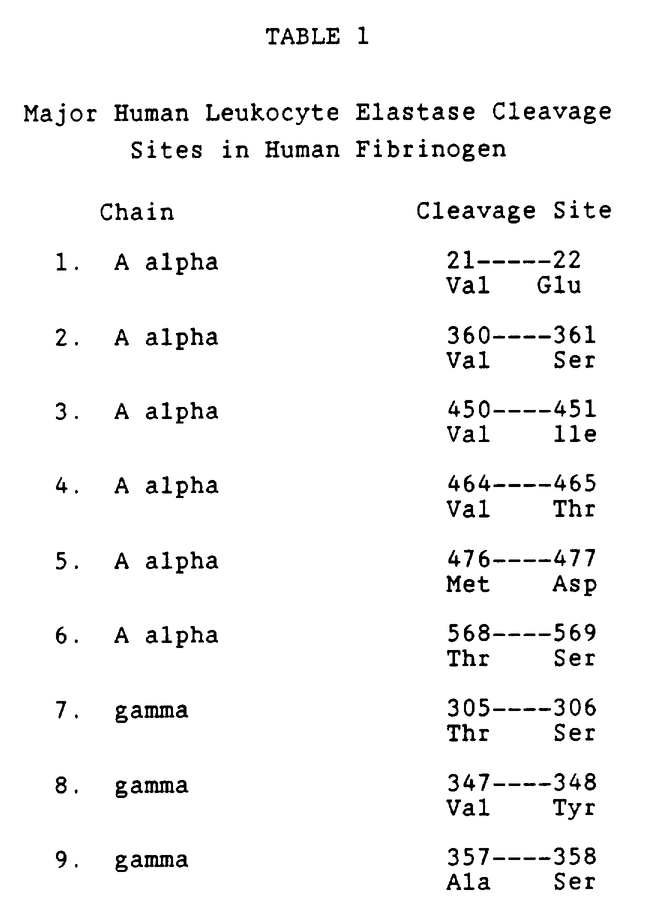

Knowing the specific elastase cleavage sites of human fibrinogen allows the synthesis of antigenic peptides and peptide probes of varying amino acid length corresponding to both the amino and carboxyl termini of the primary cleavage sites. Antigenic peptide as used herein denotes a peptide that can combine with or bind to specific antibody raised against the antigen or can induce the formation of antibody which will react with the antigen. The preferred embodiment of antigenic peptides and peptide probes includes, but is not limited to the first 15 amino acids on either the carboxyl or amino side of described enzymatic cleavage sites. It is to be understood that the embodiment of this invention includes peptides ranging in length from about 5 amino acids to about 25 amino acids or longer. All amino acid sequences depicted herein are numbered from the amino terminus to carboxyl terminus according to the scheme shown by Henschen et al., Ann. N.Y. Acad. Sci. 408:28-43 (1983). Representative carboxyl termini antigenic peptide and probe peptide amino acid sequences are shown in the following table.

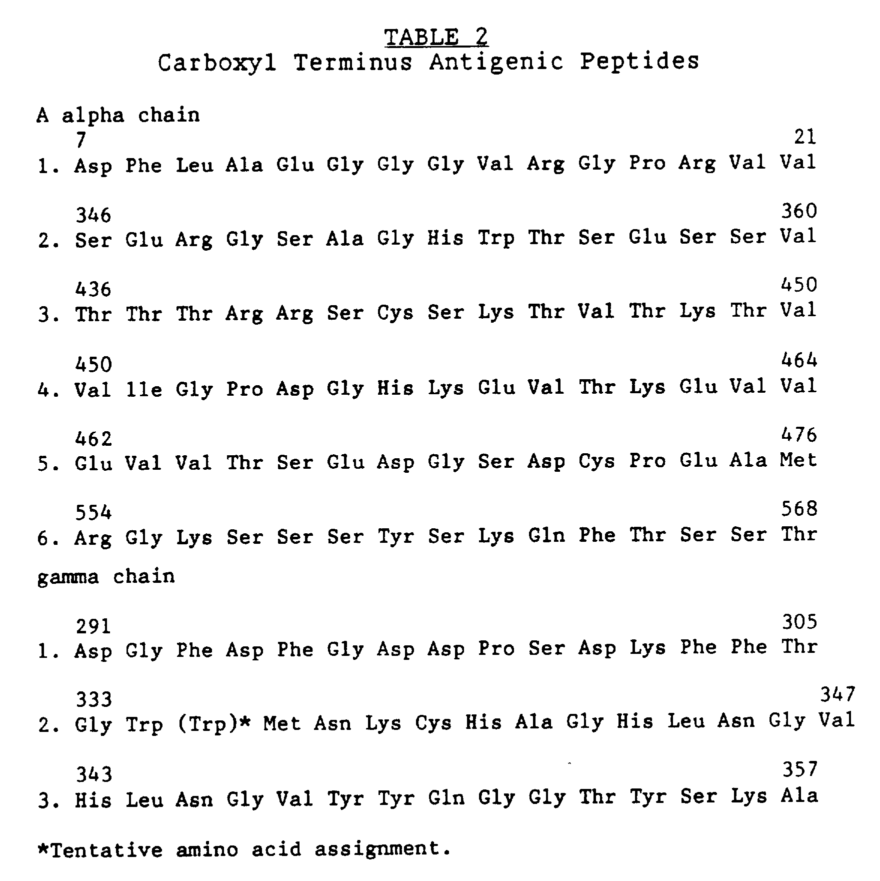

The specificity of the antigenic peptides and the peptide probes is determined by the epitope directly adjacent to the amino or carboxyl termini of the enzyme cleavage site. Epitope as used herein denotes an antigenic determinant site of known structure, amino acid sequence, which is responsible for the specificity of the antigen and can react specifically with antibody raised against the antigen which contains the epitope. The preferred embodiment of epitopes includes, but is not limited to, the first 5 amino acids on either the carboxyl or amino side of the described enzymatic cleavage sites.

Thus, in a first aspect of the present invention, there is provided an isolated and purified peptide comprising the carboxyl terminus of the primary cleavage products of human leukocyte elastase cleaved human fibrinogen which is capable of inducing specific antibodies against said cleavage products and acting as a specific probe for the detection of said antibodies, said peptide antigen having one of the following epitopes at its carboxyl terminus: 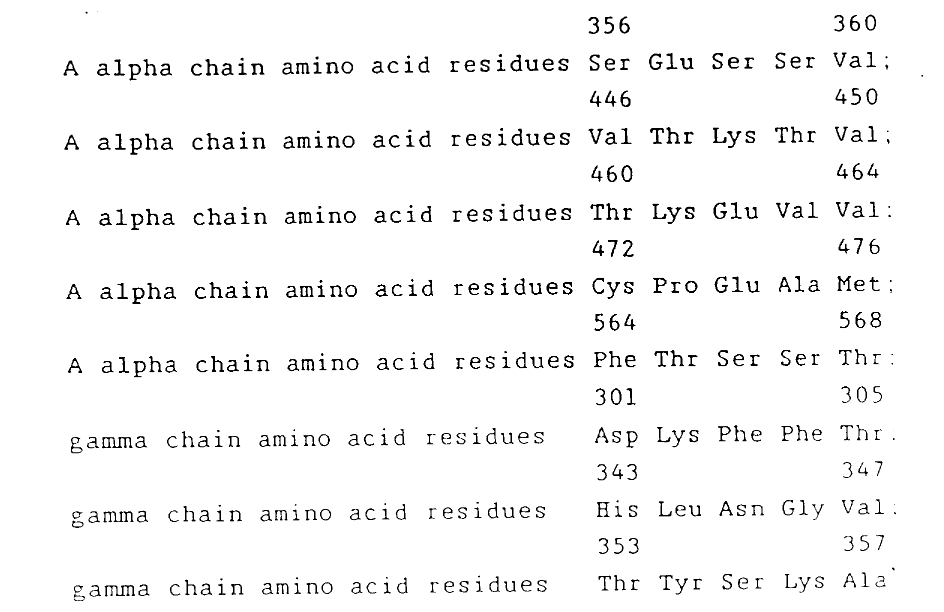 or acid addition salts thereof.

Representative amino termini antigenic peptide and probe peptide amino acid sequences are shown in the following table.

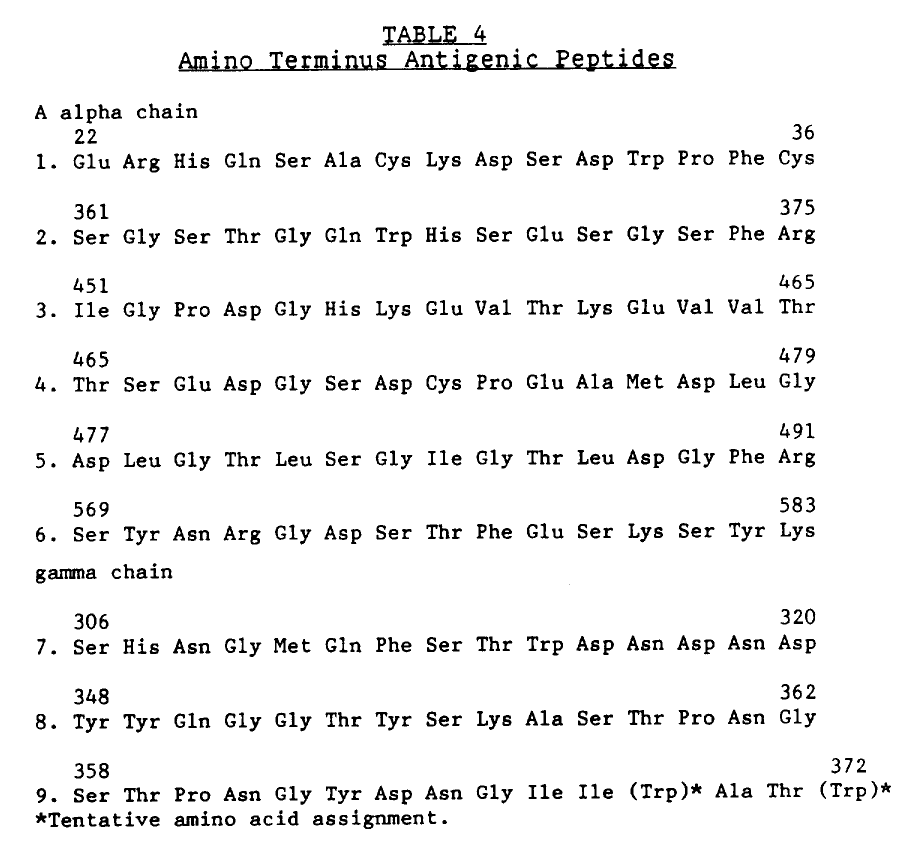

Thus, in a second or alternative aspect of the present invention, there is provided an isolated and purified peptide comprising the amino terminus of the primary cleavage products of human leukocyte elastase cleaved human fibrinogen which is capable of inducing specific antibodies against said cleavage products and acting as a specific probe for the detection of said antibodies, said peptide antigen having one of the following epitopes at its amino terminus: 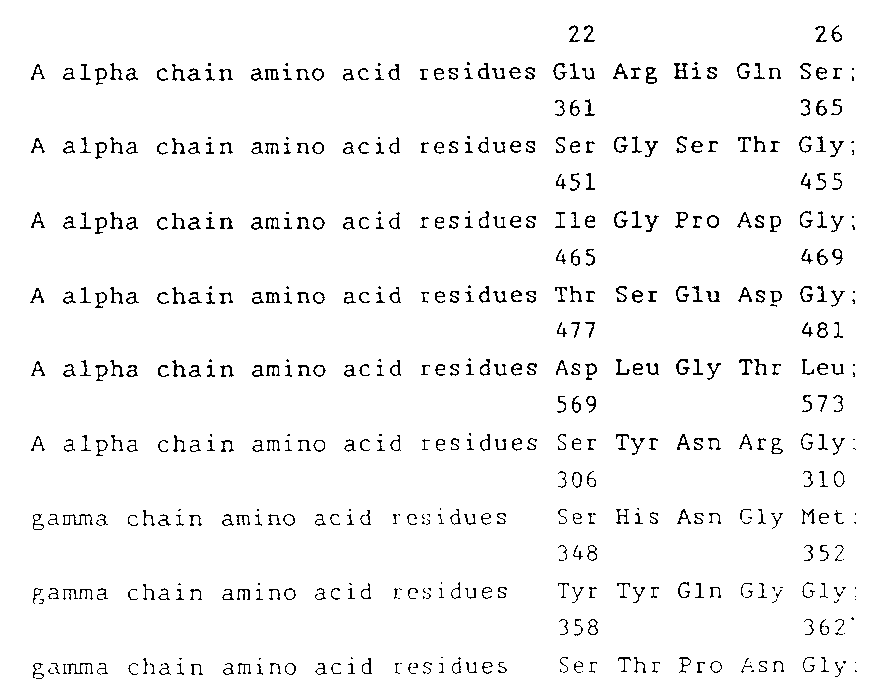 or the amide or acid addition salts thereof.

The above identified and described peptides are used as antigens to produce antibodies which react specifically with the unique amino acid sequence of the elastase cleavage product. The novel peptides are also used as specific probes or antigens for determining antiserum titer in body fluids. The various peptides can be used as specificity peptides to evaluate the specificity of the unique antibodies prepared against the various peptides.

Immunogens are prepared by attaching the individual antigenic peptides or individual epitope peptides, or any peptide containing said epitope onto an immunogenic carrier molecule capable of inducing antibody synthesis in animals. An immunogen is defined herein as a substance of sufficient size that when introduced into an animal, it stimulates the production of antibodies reactive with the specific antigen or epitope. Immunogenic carrier is defined herein as a protein or other high molecular weight compound to which an antigen or epitope is conjugated in vitro and which renders the antigen or epitope capable of stimulating an immune response. Peptides containing the specific amino acid epitope are termed herein antigenic peptides. It is to be understood that the embodiment of this invention includes the use of the defined epitopes and antigens, without the addition of immunologic carriers or any other chemical modification, as direct stimulants of antibody formation.

The peptide antigens of the present invention may be synthesized using any suitable peptide synthesis technique, such as solid phase peptide synthesis chemistry following the method of Merrifield, J. Am. Chem. Soc. 85: 2149-2154. The peptides terminating in a carboxyl group are synthesized on the standard Merrifield resin. The peptides terminating in an amide group are synthesized on a 4-methyl benzhydrylamine resin. Hydrofluoric acid cleavage of the C-terminal amino acid residue from this resin yields a peptide containing an amide terminus. The procedures are known in the art. Linking amino acids such as cysteine are added, if necessary, to either the amino or carboxyl terminus of antigen peptides either during synthesis or chemically after synthesis to provide a free sulhydryl group to facilicate coupling to an immunogenic carrier. The linking amino acid is added at the opposite end of the peptide sequence from the cleavage site to allow attachment to the carrier protein leaving the proteolytic cleavage site exposed. Internal molecular markers such as norleucine may also be added during the synthesis of antigenic peptides to evaluate the number of peptide molecules bound to the carrier. Norleucine is preferred since it is not a normal amino acid component of natural proteins. The synthesized peptides are purified by preparative reverse phase high performance liquid chromatography (HPLC) with identity and purity being established by fast atom bombardment (FAB) mass spectrometry and amino acid analysis, techinques known in the art.

The antigenic peptides are convalently coupled to high molecular weight carrier proteins which include, but are not limited to bovine serum albumin (BSA), bovine thyroglobulin (BT), keyhole limpet hemocyanin (KLH), ovalbumin (OA), and the like, with BSA and BT being preferred. The antigenic peptides are coupled to the linking amino acid, cysteine, by maleimido-NHS-ester heterobifunctional coupling reagents which include, but are not limited to, m-Maleimidobenzoyl-N-hydroxysuccinimide ester (MSB), m-Maleimidobenzoyl-sulfosuccinimide ester (Sulfo-MBS), Succinimidyl 4-(N-Maleimidomethyl)-cyclohexane-1-carboxylate (SMCC) sulfosuccinimidyl 4-(N-Maleimidomethyl)cyclohexane-1-carboxylate (Sulfo-SMCC), Succinimidyl 4-(p-Maleimidophenyl) butryate, (SMFB), sulfosuccinimidyl 4-(p-Maleimidophenyl) butyrate, (Sulfo-SMPB), with MBS and Sulfo-MBS being preferred. The carrier protein, generally BSA or BT, is prepared by dissolving about 10 mg in about 2.5 ml of about 100 mM phosphate (K⁺) buffer at about pH 7.25. All buffers used in the coupling process must be exhaustively de-gassed prior to use. To the dissolved carrier is added about 5 mg of the coupling reagent and the solution is stirred for 15 minutes to 30 minutes. The activated protein carrier is purified by size exclusion chromatography using Sephadex gel about G25, a PD10 pre-packed column (Pharmacia). The activated carrier is eluted with about 3.5 ml of about 100 mM phosphate (K⁺) buffer, pH about 5.8 into about 6 micromoles of purified lyophilized peptide for carrier-peptide linking. The mixture is maintained at about 4°C overnight and the cross-linked peptide is purified by size exclusion chromatography using Sephadex gel about G25 (Pharmacia) or dialysis and lyophilized.

The antigenic peptide-carrier conjugate is analyzed to determine the number of molecules of peptide bound to each molecule of carrier. A sample of peptide-carrier conjugate, about 0.5 mg, is hydrolyzed in about 1 ml of about 6.0 N HCl maintained at about 110°C, constantly boiling HCL in vacuo for about 24 hours. There will be one norleucine molecule in the hydrolysate for each peptide molecule bound to the carrier. An adequate immunogen, containing either a 15 amino acid peptide or a 5 amino acid peptide, will contain at least 5 moles of antigen peptide per mole of carrier.

Monospecific antibodies to the antigenic peptides are purified from mammalian antisera containing antibodies reactive against both the peptides and the carrier or prepared as monoclonal antibodies reactive with only the specific peptide using the technique of Kohler and Milstein, Nature 256:495-497 (1975). Monospecific antibody as used herein is defined as a single antibody species or multiple antibody species with homogenous binding characteristics for the relevant peptide antigen. Homogenous binding as used herein refers to the ability of the antibody species to bind to a specific antigen or epitope, such as those associated with the HLE cleavage sites of human fibrinogen, as described above. Peptide specific antibodies are raised by immunizing animals such as mice, rats, guinea pigs, rabbits, goats and horses, with rabbits and guinea pigs being preferred, with an appropriate concentration of the specific peptide-carrier complex either with or without an immune adjuvant. Preimmune serum is collected prior to the first immunization. Each animal receives between about 50 mg and about 300 mg of a single peptide-carrier complex associated with an acceptable immune adjuvant. Such acceptable adjuvants include, but are not limited to Freund's complete, Freund's incomplete, alum-precipitate, water in oil emulsion containing Corynebacterium parvum and tRNA. The initial immunization consisted of the specific peptide-carrier conjugate in, preferably, Freund's complete adjuvant at multiple sites either subcutaneously or intradermally. Each animal is bled at regular intervals, preferably weekly, to determine antibody titer. The animals may or may not receive booster injections following the initial immunization. Those animals receiving booster injections are given an equal amount of peptide-carrier conjugate in Freund's incomplete adjuvant by the same route. Booster injections are given at about three week intervals until maximal titer is obtained. At about 7 days after each booster immunization or about weekly after a single immunization, the animals are bled, the serum collected, aliquoted and stored at about -20°C.

Monoclonal antibodies (mAb) reactive with the peptide antigens of human leukocyle elastase-cleaved fibrinogen, as shown in Tables 2,3,4 and 5, are prepared by immunizing inbred mice preferably Balb/c with the appropriate peptide-conjugate. The mice are immunized by the intraperitoneal or subcutaneous route with about 0.1 mg to about 10 mg, preferably about 1 mg, of the specific antigenic peptide conjugate in about 0.5 ml buffer or saline incorporated in an equal volume of an acceptable adjuvant. Such acceptable adjuvants include, but are not limited to, Freund's complete, Freund's incomplete, alum-precipitate, water-in-oil emulsion containing Corynebacterium parvum and t-RNA, with Freund's incomplete adjuvant (IFA) being preferred. The mice received an initial immunization on day 0 and are rested for approximately 24 weeks. Immunized mice are given a booster immunization of about 1 mg of the conjugate or peptide in a buffer solution such as phosphate buffered saline by the intravenous route. At approximately day three after the booster immunization the mice are tested for peptide antibody. Lymphocytes, from antibody positive mice, preferably splenic lymphocytes, are obtained by removing spleens from immunized mice by standard procedures known to the art. Hybridoma cells are produced by mixing the splenic lymphocytes with an appropriate fusion partner under conditions which will allow the formation of stable hybridomas. Fusion partners may include, but are not limited to: mouse myelomas P3-NS1-Ag 4-1; MPC-11; S-194: NS.1 and Sp 2/0, with Sp 2/0 being preferred. The antibody producing cells and myeloma cells are fused in polyethylene glycol, about 1000 mol. wt., at concentrations from about 30% to about 50%. Fused hybridoma cells are selected by growth in hypoxanthine, thymidine and aminopterin supplemented Dulbecco's Modified Eagles Medium (DMEM) by procedures known in the art. Supernatants are collected from growth positive wells on about days 14, 18 and 21 and are screened for antibody production by a solid phase immunoradioassay (SPIRA) using the specific peptide antigens as the antigen. The culture supernates are also tested in the Ouchterlony precipitin assay to determine the isotype of the mAb. Cells from the antibody positive wells are cloned in soft agar by the technique of MacPherson, Soft Agar Techniques, in Tissue Culture Methods and Applications, Kruse and Paterson, Eds Academic Press, 1973.

Monoclonal antibodies are produced in vivo by injecting pristane primed BALB/c mice, approximately 0.5 ml per mouse, with about 2 x 10⁶ to about 6 x 10⁶ hybridoma cells, about 4 days after priming. Ascites fluid is collected at approximately 8-12 days and the monoclonal antibodies precipitated with ammonium sulfate, about 35% to about 60% of saturation, with 45% being preferred, washed and resuspended in a physiologically acceptable buffer at a pH of about 7.2. Such physiologically acceptable buffers include, but are not limited to, phosphate buffered saline, phosphate buffered saline glucose, buffered saline and the like.

In vitro production of anti-peptide mAb is carried out by growing the hybridoma in DMEM containing about 2% fetal calf serum to obtain sufficient quantities. The in vitro produced mAb is purified by the same procedure as that for the ascites fluid. The monoclonal antibodies are further purified by affinity chromatography using a protein A Sepharose matrix and the technique of Ey et al., Immunochemistry 15:429-436 (1978) for IgG antibodies or a similar technique for other antibody isotypes. The purified monoclonal antibody, is neutralized with about 1M phosphate buffer at about pH 8.0 and stored as above.

Antibody titers of animal sera or monoclonal antibodies, antibody sensitivity, antibody specificity and the presence of and concentration of unknown antigens in body fluids are determined by various serological or immunological assays including precipitation, passive agglutination, enzyme-linked immunosorbent antibody (ELISA) technique, and radioimmunoassay (RIA) with RIA being preferred. Titer is defined herein as a measure of the concentration of antibody in a serum sample. The antibody titer of a given serum sample is related to the affinity of the antibody for the antigen. A serological or immunological assay is defined as a method of determining a specific component of a mixture or a specific amount of a known substance in which specific antigens or antibodies are used for the determination. Each assay includes the appropriate controls. The RIA is carried out in an assay buffer which may include, but is not limited to, buffered saline, phosphate buffer, phosphate buffered saline, Dulbecco's phosphate buffered saline, Dulbecco's calcium-and-magnesium-free buffered saline with Dulbecco's calcium-and-magnesium-free phosphate buffered saline (GIBCO) being preferred. The assay buffer may be used alone or preferably supplemented with about 0.1% gelatin, about 0.01% thimerasol and 1.0 mM ethylenediamine-tetraacetic acid (EDTA). Antiserum or monoclonal antibodies are prepared for antibody titer determination by dilution in the assay buffer. The initial dilutions are generally 1:1,000, 1:5,000 and 1:10,000.

Antigenic probes useful for antibody binding and titer determination include either synthesized or native specific antigen peptides containing the specific epitope. The peptides used as antigenic probes may contain only the specific amino acid epitope sequence or the amino acid antigenic sequences as depicted in Tables 2, 3, 4 and 5, modifications thereof, or they may include longer peptide units of the A alpha or gamma chain of fibrinogen which include the specific antigenic or epitope sequences and modifications. The antigenic probes may also include native peptides or synthesized peptides of as many as about 20 to about 30 amino acids but which also include the specific antigen or epitope sequence. Synthesized peptide probes are produced by the Merrifield technique, supra. Probes are selected or designed to be easily attached to a label or a tracer such as enzymes, fluorscent dyes, radioisotopes or haptens. The tracer or label allows for quantification of the serologic reaction. Synthesized peptides are designed to contain one or more additional amino acids which will allow the coupling of the label to the antigenic probe. Radioisotopes are the preferred label or tracer for the embodiments of this invention. Probe peptides are labeled with tritium (³H), ¹⁴carbon (¹⁴C), ¹⁵iodine (¹⁵I) or ¹³¹I, with ¹⁵I being preferred. To facilitate radioiodination each synthesized peptide is designed to contain one or more tyrosine residues, which may be part of the natural sequence, to allow coupling of the label to the antigenic probe. Radioiodination is accomplished using the chloramine T, iodogen or lactoperoxidase process with the chloramine T process being preferred. The labeled probed peptides are purified by reverse phase HPLC, size exclusion chromatography or ion exchange chromatography with HPLC being preferred. The amount of radioactivity per peptide is determined by procedures known in the art.

The radioactive probe is diluted in assay buffer to yield about 10,000 counts per minute (cpm) to about 30,000 cpm per aliquot, with about 20,000 cpm being preferred. The appropriate concentration of the specific probe is contacted with the diluted antibody. All determinations are made in duplicate. Following the contacting of the diluted antibody with the labeled probe, the mixture is incubated at about 4°C for about 10 to about 20 hours. Non-antibody-bound radioactive probe is removed from the reaction mixture by chromatographic, precipitation, immunoprecipitation or adsorption techniques with adsorption of the unbound probe onto dextran-coated activated charcoal being preferred. The dextran-coated charcoal is prepared by suspending activated charcoal, USP, at about 3% (w/v) in 10 mM phosphate buffer, pH 7.5, containing about 0.25% (w/v) dextran, about 60,000 to about 90,000 average mol. wt., with about 70,000 average mol. wt. being preferred. The dextran-charcoal mixture is allowed to stand overnight, sedimented by centrifugation, washed once in dextran-containing phosphate buffer as above and centrifuged again. The dextran-coated activated charcoal is resuspended in the same buffer about to a 3% concentration. Immediately before use in the assay, the dextran-coated activated charcoal is diluted about 10-fold in Dulbecco's PBS and an amount sufficient to bind free probe is added to each sample. After a brief incubation period in a slurry of ice in water, the charcoal is sedimented by centrifugation and the supernatant fluid collected and counted in a gamma counter. Each assay includes charcoal-free controls, to which about 1 ml of PBS is added, for determination of total counts, and antibody-free controls for determination of non-specific binding. Percent specific binding at each antiserum dilution is determined by subtracting the value for non-specific binding from the value of antiserum binding and then dividing by the total counts per minute.

Sensitivity of the antisera is also determined by serologic assays, with RIA being preferred. The antiserum is diluted in an appropriate buffer, such as assay buffer, such that it binds about 15 percent to about 40 percent of the total radioactive probe, with about 25 percent being preferred. The same buffer is used for all dilutions. The appropriate peptide antigen, complementary to the specific antibody, is diluted in assay buffer to approximate concentrations to yield about 1 pmole, about 0.1 pmole and about 0.01 pmole of peptide per assay aliquot. Complementary as used herein is defined as the specific amino acid sequence of an epitope that will specifically bind to an antibody raised specifically against that epitope resulting in an antiepitope antibody. The radioactive probe, ¹⁵I-labeled peptide complementary to the specific antibody, is diluted in assay buffer to yield about 10,000 cpm per assay aliquot. The assay includes antibody-free samples for determination of non-specific binding, and controls containing antibody and probe without unlabeled peptide, to determine the control level of probe binding. All determinations are made in duplicate.

Following the contacting of the diluted antibody with the labeled probe the mixture is incubated at about 4°C for about 10 to about 20 hours. Antibody-bound radioactive probe is removed from unbound probe by chromatographic, precipitation, immunoprecipitation or adsorption techniques with adsorption of the unbound probe onto dextran-coated activated charcoal being preferred, as described above. Diluted dextran-coated activated charcoal is added to each sample. After a brief incubation period in a slurry of ice in water, the charcoal is sedimented by centrifugation and the supernatant fluid collected and counted in a gamma counter. The presence of non-labeled peptide inhibits the binding of labeled probe. The inhibition increases as the concentration of non-labeled peptide increases. The extent of inhibition at each level of peptide is calculated as the percent of the specific binding observed in the absence of unlabeled peptide. The sensitivity of antisera are compared by determining for each anti serum the 50% inhibitory concentration (IC₅₀) of the specific peptide and then comparing that to the value for the other sera. The IC₅₀ as used herein is defined as the concentration of antigen producing 50 percent inhibition of probe binding.

Specificity of the individual antisera is also determined by serologic assays, with RIA being preferred, as described above. The antiserum is diluted in an appropriate buffer, such as assay buffer, to a concentration that binds about 15 percent to about 40 percent of the total radioactive probe, with about 25 percent being preferred. Various length probe peptides, which may contain at least the five amino acids adjacent to the amino or carboxyl cleavage sites of the Aa or gamma chain, including as a standard the specific peptide antigen complementary to the antiserum are diluted in assay buffer to yield amounts ranging from about 10⁻¹³ moles to about 10⁻⁶ moles in the assay aliquot. Controls will include probes which do not contain the specific epitope. The radioactive probe, ¹⁵-I-labeled peptide complementary to the specific antibody is diluted in assay buffer to yield about 5,000 cpm to about 25,000 cpm with a range of about 10,000 cpm to about 20,000 cpm being preferred per assay aliquot. The volume of each reactant in an assay may range from about 1 ml to about 1 ml, with about 100 ml being preferred. Specificity is determined by either an equilibrium assay or a non-equilibrium assay. In an equilibrium assay the specificity probes are diluted and contacted with a constant amount of antibody and further contacted with a constant amount of complementary labeled probe and incubated for about 10 to about 20 hours. In a non-equilibrium assay the specificity probes are diluted and contacted with a constant amount of antibody and incubated for about 2 to about 20 hours. After the initial incubation, the labeled probe is added and the samples are incubated for a second time for about 30 minutes to about 4 hours. The equilibrium assay is the preferred assay. The assay includes antibody-free controls to evaluate non-specific binding and controls containing antibody and probe without unlabeled peptide to determine the control level of probe binding.

Following the contacting of the antibody and the specificity probe and the subsequent addition of the labeled probe, the mixture is incubated at about 4°C for about 10 to about 20 hours. Antibody-bound radioactive probe is separated from unbound probe by chromatographic, precipitation, immunoprecipitation or adsorption techniques, with adsorption of the unbound probe onto dextran-coated activated charcoal being preferred, as described above. After a brief incubation period in a slurry of ice in water, the charcoal is sedimented by centrifugation and the supernatant fluid, containing the antibody-bound probe, is collected and counted in a gamma counter. All techniques are known to the art.

The control level of probe binding is determined and the extent of inhibition at each concentration of specificity probe is calculated from the percent of the specific binding observed in the absence of specificity probe. The specificity of each antiserum is measured by comparing the levels of the various specificity probes which yield 50 percent inhibition of labeled probe binding. For a highly specific antiserum, much higher levels of specificity probe than the specific one complementary to the antiserum will be required to yield 50 percent inhibition.

The monospecific antibodies, as identified and characterized above, are used to assay for the formation of the complimentary elastase cleavage product antigens or epitopes in whole blood or other body fluids, peritoneal fluid, sputum or broncheoalveolar lavage fluid. The assay for cleavage products is dependant upon the presence of leukocyte elastase in the sample. The preferred elastase is neutrophil elastase, but the embodiment of the invention includes all leukocyte elastases. The elastase may be released from leukocytes present in the sample fluid, as described below, or may be added to the assay by adding viable leukocytes or by the addition leukocyte extracts or purified elastase. The disease state will generally determine what fluid samples are necessary for the detection of elastase cleavage products. Blood and broncheovalveolar lavage fluid are the preferred fluids for following disease states; pulmonary emphysema, chronic bronchitis, cystic fibrosis, chronic obstructive pulmonary disease, bronchiectasis, adult respiratory distress syndrome, myelogenous leukemia and infantile respiratory distress syndrome. Blood and synovial fluid are the preferred fluids for the following diseases; arthritis and gout. The fluids of choice for glomerulonephritis is blood, urine and peritoneal fluid.

Aliquots of freshly-drawn blood or other body fluids including lavage washes which contain cells, are treated with an anticoagulant. Such anticoagulants include, but are not limited to, ammonium or potassium oxalate, disodium citrate, sequestrene, fluoride and heparin, with heparin being preferred. The samples are incubated in the presence of compounds capable of perturbing the cell membranes of leukocytes resulting in the release of intracellular elastase. Perturbation as used herein is defined as a variation in or alteration of or deviation from the normal structural integrity of leukocyte cellular and vesicular membranes. Perturbating agents allow the release of intracellular enzymes, such as elastase, from intact cells by membrane alteration resulting in exocytosis or the like or may lyse the membranes and release the enzymes. Agents capable of membrane perturbation include, but are not limited to, zymosan, antigen-antibody complexes, complement C5a, N-formyl-L-methionyl-L-leucyl-L-phenylalanine (FMLP), phorbol-12-myristate-13-acetate (PMA) plus cytochalasin B, n-formyl-L-norleucyl-L-leucyl-L-phenylalanine (FNLP) plus cytochalasin B and calcium ionophore A23187, platlet acavating factor or any membrane perturbing agent which can alter the membranes of leukocytes resulting in the release of intracellular enzymes. The preferred agent for membrane perturbaton is the calcium ionophore A23187 in concentrations ranging from about 75 mM to about 300 mM per aliquot. Prior to adding to the blood or body fluid, the calcium ionophore is dissolved in DMSO with the final concentration of DMSO in blood remaining constant at about 0.2%. Following incubation at about 37°C for about 25 minutes, the blood or body fluid is centrifuged at about 2,000 x g and the plasma or cell free body fluid is collected. The unique elastase cleavage products of fibrinogen in plasma or acellular fluid can be assayed by the above procedure or the protein can be removed from the plasma by precipitation with chilled ethanol at a ratio of about 3:1 ethanol to plasma or fluid. The plasma-ethanol or fluid-ethanol mixture is centrifuged at about 3,000 x g, the supernatant fluid collected and evaporated to dryness in a Speed-vac concentrator. The treated plasma or fluid is reconstituted to original volume with distilled water and samples are assayed for the presence of the unique elastase cleavage products of fibrinogen by an assay procedure capable of detecting the specific antigens or epitopes. As the concentration of calcium ionophore A23187 increases the amount of cleavage product increases until it reaches a maximum at an ionophore concentration of about 150 mM.

Fluid samples which do not contain neutrophils can also be assayed in a similar manner. These samples may be treated with a standard concentration of neutrophils and then treated with a perturbing agent or they may be treated with a standard amount of purified elastase. The fluids, cells and elastase in most situations would be homologous.

Elastase cleavage products can be identified and the concentration determined by a variety of immunoassays, which include but are not limited to: immunoradiometric assays, employing labelled antibodies, Miles and Hales, Nature 219: 186- (1968) or a sandwich varient according to Addison and Hales, Hormone Meta. Res. 3:59- (1971); enzyme immunoassays, employing competitive assays with antigen-enzyme conjugates according to the method of Van Weeman and Schours, F.E.B.S. Lett. 15: 232- (1971) or enzyme-labelled antibody assays according to the procedure of Envall and Perlmann, Immunochem. 8:871- (1971) or a variant of the enzyme-labelled antibody assay which employs detection by a second antibody according to the process of Hammarstrom et al., Proc. Natl. Acad. Sci. USA 72: 1528- (1975) or non-competitive sandwich (ELISA) methods, see Nakamura et al. in Handbood of Experimental Immunology, Vol. 1, Immunochemistry, D.M. Weir, ed. 4th Edition (1986), Blackwell Scientific Publications, Oxford, pp. 27.1-27.20: or labelled antigen (RIA) as described above or any other assay which will determine antigen concentration. The preferred assay is the RIA as generally described above and in more detail hereinafter. The total volume of the assay fluid may range from about 5 ml to about 1 ml with about 100 ml to about 500 ml being preferred. Standard solutions of known or unknown cleavage products are prepared in assay buffer, as described above, in the range of about 1 x 10⁻¹⁰ M to about 2 x 10⁻⁸ M which represents amounts of peptide ranging from about 0.01 picomoles to about 2.0 picomoles in an appropriate volume, about 100 ml of buffer. The standard solutions or dilutions of unknown samples are contacted with an antibody solution. The antibody solution may contain one or more monospecific antibodies specifically reactive with the antigens listed in Tables 2, 3, 4 and 5 or antibodies reactive with any epitope listed in the Tables on any length fragment of fibrinogen. The antibody solution is generally at a concentration sufficient to give a positive reaction, usually about a 1:1000 dilution of serum in an appropriate buffer such as assay buffer. The samples containing antigen and antibody are mixed and a labeled probe complementary to the specific antibody or antibodies is added. A radioactive probe or probes will be diluted in an appropriate buffer, assay buffer, to yield about 10,000 to about 25,000 cpm per about 100 ml of buffer. Each assay includes antibody-free controls to measure non-specific binding and controls containing antibody plus probe without added standard or unknown to determine the control level of peptide binding.

Following the contacting of the diluted antibody with the labeled probe, the mixture is incubated at about 4°C for about 10 to about 20 hours or at about 37°C for about 10 minutes to about 2 hours. Antibody-bound radioactive probe is separated from unbound radioactive probe by chromatographic, precipitation, immunoprecipitation or adsorption techniques with adsorption of the unbound probe onto dextran-coated activated charcoal being preferred, as described above. Diluted dextran-coated activated charcoal is added to each sample. After a brief incubation period in a slurry of ice in water, the charcoal is sedimented by centrifugation and the supernatant fluid collected and counted in a gamma counter. Each assay includes charcoal-free controls for determination of total counts and antibody-free controls for determination of non-specific binding. The antibody-free control and the zero-peptide antibody control are counted in a gamma counter to determine the 0% bound and 100% bound values respectively. The assay standards are counted and related to the antibody control to determine the percent probe bound, and a standard curve is generated. When the percent bound is plotted as a function of the logarithem of the amount of peptide in the standard, a sigmoidal curve is generated which is close to linear between the limits of about 80% of control to about 20% of control. Unknowns are counted, their percent of control calculated, and they are compared to the standard curve to determine the amount of peptide present in the sample. Only those unknowns counting between about 80% and about 20% of control binding are considered valid.

The above defined assay technology is used to monitor the activity of human elastase inhibitors on human leukocyte elastase activity in human and primate blood. Generally an elastase inhibitor is combined with whole blood or given to primates or humans and the effect of leukocyte elastase on fibrinogen is determined. Replicate aliquots of freshly-drawn heparinized whole human blood are prepared with concentrations of elastase inhibitor ranging up to about 300 mg/ml. Following a brief pre-incubation with the inhibitor, a membrane perturbator, such as calcium ionophore A23187, is added at a concentration of between about 75 mM and about 300 mM. Non-membrane perturbator controls containing blood and perturbator-only controls are included to measure the extent of uninhibited peptide generation. All assay samples are incubated at about 37°C for about 25 minutes. The plasma is then prepared and assayed for fibrinogen cleavage products as described above. Elastase inhibitors are capable of inhibiting the generation of fibrinogen cleavage products and the levels of inhibition are easily detected using this novel assay system.

In vivo inhibition of fibrinogen cleavage products following treatment of primates with an elastase inhibitor is evaluated. Blood or fluid samples are collected both before and after treatment with either an elastase inhibitor or saline. Each heparinized blood sample is divided into about 4 aliquots (about 1 ml) and processed as described above. Treatment of an animal with an elastase inhibitor causes a marked reduction in the amount of elastase cleavage product produced.

The ability of the novel assay to determine the presence of the unique fibrinogen cleavage products and to determine the relative amounts of these products is evaluated with blood from individuals genetically deficient in alpha 1-proteinase inhibitor (α1Pi), a normal serum elastase inhibitor. Individuals deficient in α1Pi, exhibit the PiZZ phenotype, and produce less than normal levels of circulating alPi which is a natural inhibitor of leukocyte elastase, Janoff, Am. Rev. Respir. Dis. 132: 417-433 (1985). Consequently individuals exhibiting the PiZZ phenotype would not have the capacity to inhibit elastase activity and they should have increased fibrinogen cleavage products when assayed by the above procedure. When heparinized blood is collected from individuals who possess the PiZZ phenotype and processed as described above and levels of specific cleavage peptide antigen are measured, they are higher than normal volunteers.

The following examples illustrate the present invention without, however, limiting the same hereto.

### EXAMPLE 1

### Primary Cleavage of Human Fibrinogen By Human Leukocyte Elastase

Purified human fibrinogen (Sigma) was combined with human neutrophil elastase (Elastin Products), at the ratio of 200 moles fibrinogen/mole human neutrophil elastase in Tris-buffer, pH 7.0 in a final volume of 1.0 ml. Samples of the reaction mixture were incubated at 37° for 5, 15, 30 45 and 60 minutes. The reaction was stopped by the addition of 3 volumes of ice-cold ethanol (3°C) and incubated at that temperature for 30 minutes. Following incubation the samples were centrifuged at 3,000 x g at 3°C for 20 minutes, the supernatant fluid was removed and placed in clean tubes and the samples concentrated to dryness in a Speed-Vac overnight. Each sample was reconstituted in high performance liquid chromatography HPLC buffer consisting of a mixture of water and acetonitrile and chromatographed on a Dupont-ZorbaxT C-18 column (0.4 x 25 cm). A linear gradient was developed over a 75 minute interval in H₂O:acetonitrile:trifluoroacetic acid, 95:5:0.2 v/v/v, to H₂O:acetonitrile:trifluoroacetic acid, 60:40:0.2 v/v/v. Peptide peaks emerging from the column were collected, rechromatographed to prove purity and subjected to N-terminal amino acid sequence analysis on an Applied Biosystems Protein Peptide Sequencer, Model 470A, following the manufactures procedures.

A 15 minute incubation time allowed the best resolution of fibrinogen cleavage products. Eight major peaks and nineteen minor peaks were identified. The major peaks represent the primary cleavage products and the minor peaks represent the minor or secondary cleavage sites. Amino acid sequence analysis of the major peptide peaks allowed the identification of nine elastase cleavage sites in human fibrinogen. Six of the cleavage sites reside in the A alpha chain while three sites are located in the gamma chain, see Table 1.

### EXAMPLE 2

### Synthetic Peptide Antigens

Knowing the specific elastase cleavage sites on both the A alpha and gamma chain, see Example 1, of human fibrinogen allowed the identification of antigenic peptides and peptide probes representing the amino and carboxyl termini adjacent to the cleavage sites. The specific amino acid sequences associated with the carboxyl termini are illustrated in Tables 2 and 3 while the sequences associated with the amino termini are illustrated in Tables 4 and 5.

Peptide antigens and peptide probes were synthesized using t-Boc chemistry on a SAM TWO Peptide Synthesizer (Biosearch, Inc.) and an Applied Biosystems (Model 430) peptid synthesizer. The peptides terminating in a carboxyl group were synthesized on the standard Merrifield resin. The synthesis procedure is described in detail in the SAM TWO Peptide Synthesizer Operator's Manual (Biosearch, 1985). Cleavage of the peptide from the resin was accomplished by treatment with hydrogen fluoride in a Protein Research Foundation hydrogen fluoride apparatus following procedures outlined in the SAM TWO Operator's Manual. Correct molecular ions were obtained by fast atom bombardment spectral analysis for each peptide. The peptides were purified by HPLC using an alkyl silane substrate with 18 carbon atoms (C18) as described in the SAM TWO Operator's Manual. Purity of the individual peptides was assessed by reversed phase HPLC and amino acid analysis, again following set procedures as described in the SAM TWO Operator's Manual. Correct molecular ions were obtained by fast atom bonbardment mass spectral analysis for each peptide. The primary antigen associated with the carboxyl terminus for the Val (21) Glu (22), see Table 1, elastase cleavage site of the A alpha chain, Gly(17)-Pro-Arg-VAl-Val(21), is synthesized with two additional amino acid residues. The cysteine-norleucine is attached to the Aa 17-21 antigen to give the following antigen: Cys-Nle-Gly-Pro-Arg-Val-Val. The integrity of the carboxyl group on the terminal valine residue was unaltered. The cysteine is a linking amino acid because it allows the antigen to be linked or attached to an immunogenic carrier. The norleucine was added as an internal marker to determine the actual number of antigen molecules attached to a single immunogenic carrier.

### EXAMPLE 3

### Attachment Of Antigen to Carrier

Attachment of the antigenic peptide, from Example 2, to the carrier protein was carried out according to a modification of the method of Lerner et al., Proc. Nat. Acad. Sci. USA 78: 3403-3407 (1981) using either of the heterobifunctional coupling reagents, MBS or Sulfo-MBS (Price Chemical Co.). The peptide antigen is attached to the carrier bovine serum albumin (BSA), by combining 10 mg BSA dissolved in 2.5 ml phosphate buffer, 20 mM, pH7.0 with 4.2 mg Sulfo-MBS and incubating for 30 minutes at room temperature with stirring. The carrier-coupling reagent mixture was then applied to a PD-10 disposable Sephadex G-25 column (Pharmacia) which had been equilibrated with de-gassed 50 mM phosphate buffer, pH 6.0. The material which eluted from the column during sample application corresponded to the void volume of the column and was discarded. A small vial containing 6 micromoles of the purified, lyophilized peptide antigen was placed under the column outlet and the activated albumin was eluted into the vial with an additional 3.5 ml of the pH 6.0 buffer. The peptide antigen-activated carrier complex was allowed to react overnight. The antigen-carrier complex was purified by a second gel filtration as previously described, dialyzed against three changes of distilled water and lyophilized.

The degree of coupling was determined by amino acid analysis following hydrolysis of 0.5 mg of the lyophilized conjugate in 1 ml of 6.0 N HCl maintained at 110°C for 24 hours. The sample was subjected to amino acid analysis with ninhydrin detection. The analysis showed that there were 11 moles of antigen peptide per mole of BSA.

### EXAMPLE 4

### Production of Monospecific Antibody

New Zealand White Rabbits and Hartley outbred guinea pigs were immunized with the immunogen of Example 3. Multiple intradermal (ID) injections based on the method described by Vituakaitis, Meth. Enzymol. 73: 46-52 (1981) were employed. The initial immunizations employed 1 mg of the immunogen conjugate per ml saline homogenized with 1 ml Freund's Complete adjuvant. Rabbits received a total of 2 ml divided over 20 ID sites while guinea pigs received 1 ml. Animals were boosted 30 days later. Rabbits received 0.5 mg immunogen per ml saline, homogenized with 1 ml Freund's Incomplete adjuvant while guinea pigs received 0.25 mg immunogen. The boost was divided between an intramuscular and subcutaneous site. Frequent analytical bleeds were taken in order to assess the titer and sensitivity of the antisera. The antibody titer was allowed to fall almost to basal levels before re-boosting. Ten days after the final booster immunization, the animals were bled, the serum was collected and the serum was frozen and stored at -70°C.

### EXAMPLE 5

### Synthetic Probes

Antigen probes to determine antibody specificity and to evaluate the presence of and amounts of fibrinogen cleavage products were synthesized by the process of Example 2. Antigenic probes used to determine the presence of and amounts of cleavage products were designed to include a tyrosine residue at the terminus distal to the epitope so that the probe could be coupled to ¹⁵I. The initial probe used to determine antibody titer consisted of the first 21 amino acids of the A alpha chain plus an amino terminal tyrosine residue.

1
2N-Tyr-Ala-Asp-Ser-Gly-Glu-Gly-Asp-Phe-Leu-Ala-Glu-Gly-Gly-
21
Gly-Val-Arg-Gly-Pro-Arg-Val-Val-COOH

Specificity probes used to evaluate antibody specificity and to demonstrate that specificity resided in the Aα 17 to 21 amino acid sequence included the following: 
1
H2N-Ala-Asp-Ser-Gly-Glu-Gly-Asp-Phe-Leu-Ala-Glu-Gly-Gly-
21
Gly-Val-Arg-Gly-Pro-Arg-Val-Val-COOH
5
H2N-Glu-Gly-Asp-Phe-Leu-Ala-Glu-Gly-Gly-Gly-Val-Arg-Gly-Pro-
 
21
Arg-Val-Val-COOH
9                                                                    21
H2N-Leu-Ala-Glu-Gly-Gly-Gly-Val-Arg-Gly-Pro-Arg-Val-Val-COOH

12                                                   21
H2N-Gly-Gly-Gly-Val-Arg-Gly-Pro-Arg-Val-Val-COOH
14                                       21
H2N-Gly-Val-Arg-Gly-Pro-ARg-Val-Val-COOH
15                                  21
H2N-Val-Arg-Gly-Pro-Arg-Val-Val-COOH
17                    21
H2N-Gly-Pro-Arg-Val-Val-COOH
17                    21
H2N-Gly-Pro-Arg-Val-Val-CONH2

1
H2N-Ala-Asp-Ser-Gly-Glu-Gly-Asp-Phe-Leu-Ala-Glu-Gly-Gly-
                                          22
Gly-Val-Arg-Gly-Pro-Arg-Val-Val-Glu-COOH
1
H2N-Ala-Asp-Ser-Gly-Glu-Gly-Asp-Phe-Leu-Ala-Glu-Gly-Gly-
                              20
Gly-Val-Arg-Gly-Pro-Arg-Val-COOH
1
H2N-Ala-Asp-Ser-Gly-Glu-Gly-Asp-Phe-Leu-Ala-Glu-Gly-Gly-
                          19
Gly-Val-Arg-Gly-Pro-Arg-COOH
1
H2N-Ala-Asp-Ser-Gly-Glu-Gly-Asp-Phe-Leu-Ala-Glu-Gly-Gly-
                     18
Gly-Val-Arg-Gly-Pro-COOH
 
1
H2N-Ala-Asp-Ser-Gly-Glu-Gly-Asp-Phe-Leu-Ala-Glu-Gly-Gly-
16
Gly-Val-Arg-COOH
 Radioiodination of the assay probe was accomplished by reaction with Chloramine T. The peptide probe was dissolved in water at a concentration of 220 mg/ml. A 50 ml volume of this solution (containing 11 mg) was added to 10 ml of 0.5 M phosphate (K+) buffer and then combined with 2mCi of ¹⁵I Na and 10 ml (0.1 mg/ml) freshly prepared Chloramine T in water. The mixture was allowed to react for 30 seconds and the reaction was stopped with 10 ml of 1 mg/ml NaI plus 1 mg/ml sodium thiosulfate. The radioiodinated probe was purified by HPLC using a Supelco C-8 column (0.4 X 25 cm). The iodinated probe was eluted by a 30 minute 2% per-minute gradient of 90% eluant A-10% eluant B to 30% eluant A-70% eluant B at a flow rate of 1 ml per minute. Eluant A consisted of 0.1% trifluoroacetic acid in water and eluant B consisted of 0.1% trifluoroacetic acid in acetonitrile.

### EXAMPLE 6

### General Immunoassay Protocol

The assay was conducted in a total volume of 300 ml of Dulbecco's calcium-and-magnesium-free phosphate buffered saline supplemented with 0.1% gelatin, 0.01% thimerasol and 1.0 mM EDTA. To 100 ml of buffer or sample were added 100 ml each of antiserum and radioactive probe diluted in the same buffer. The dilution of radioactive probe was prepared such that 15,000 to 20,000 cpm are added to each sample or control. The assay was incubated overnight at 4°C and terminated by the addition of 0.3% dextran-coated charcoal. After sedimentation of the charcoal by centrifugation, the supernatant fluid was decanted and the amount of radioactivity determined.

Antiserum titers were determined in this protocol by varying the dilution of the antiserum added in the 100 ml volume. The antibody dilution used in competition experiments was selected to yield approximately 30% binding of the radioactive probe. Antibody sensitivities were determined using samples containing different known amounts of the Aα 1-21 peptide. Specificities were determined by comparing the displacement curve generated by Aα 1-21 peptide to those generated by putative crossreactive peptides. The concentration of peptide in unknown samples was determined by relating the percent of control antibody binding of probe obtained in the presence of sample to a standard curve generated using known concentrations of peptide.

### EXAMPLE 7

### Determination Of Antibody Binding

The antibody titer for the most active rabbit antisera, Example 4, against the peptide antigen of Example 1 was determined using a radioimmunoassay. The antisera was diluted in assay buffer, see Example 6, with dilutions ranging from 1:3,000 to 1:30,000 per 100 ml. The diluted antibody was contacted with Aα 1-21 radiolabeled probe, see Example 3, containing the appropriate antigenic peptide sequence, Gly-Pro-Arg-Val-Val, 100 ml. The radioactive probe was diluted in assay buffer to yield approximately 13,000 cpm per 100 ml aliquot. The assay volume was made up to 300 ml by the addition of 100 ml assay buffer. All determinations were made in duplicate.

After overnight incubation at 4°C, antibody-bound and unbound radioactive probe were separated by adsorption of the unbound probe onto dextran-coated charcoal. Dextran-coated charcoal was prepared by suspending activated charcoal, USP, at a 3%, w/v, concentration in 10 mM phosphate buffer, pH 7.5, containing 0.25%, w/v, T-70 dextran, 70,000 average molecular weight (Pharmacia). The mixture was allowed to stand overnight, sedimented by centrifugation, washed once in dextran-containing phosphate buffer as above, then resuspended to a 3% concentration in the dextran-containing assay buffer. Immediately prior to use in the assay, the dextran-coated charcoal was diluted 10-fold in Dulbecco's PBS and 1 ml is added to each assay tube. After an incubation period of 30 minutes in an ice/water slurry, the charcoal was sedimented by centrifugation at 3,000 x g for 10 minutes and the supernatant fluid was decanted and counted in a gamma counter. The assay included charcoal-free controls, to which 1 ml PBS was added, for determination of total counts and antibody-free controls for determination of non-specific binding. The percent specific binding at each antiserum dilution was determined by subtracting the antibody-free, or non-specific binding value from each value for antibody binding, and dividing that by the total counts in the system. The results are shown in the following table.

**TABLE 6**

| Percent Specific Antibody Binding | | |
|---|---|---|
| Antiserum Dilution | ¹⁵ I-CPM | Percent Specific Binding |
| 3,000 | 3,700 | |
| | 3,828 | 26.5 |
| 15,000 | 1,134 | |
| | 1,109 | 6.5 |
| 30,000 | 717 | |
| | 787 | 3.7 |
| Total | 12,980 | |
| | 13,454 | -- |
| Non-specific | 243 | |
| | 273 | -- |

Rabbit antiserum designated number 20, at a final dilution of 1:3000 binds 26 percent of the total radioactive probe. This antibody concentration is more than sufficient for determining the presence of specific cleavage peptides.

### EXAMPLE 8

### Determination Of Antibody Sensitivity

Antibody sensitivity was determined by evaluating the ability of various concentrations of unlabelled probe to inhibit binding to the radioactively labelled probe. The antiserum, Example 4, was diluted in the assay buffer, Example 6, to a concentration of 1:1,000 and 100 ml was used in each sample of the assay. The unlabelled probe, Aα 1-21, was diluted in assay buffer to give final concentrations of 1.0, 0.1, 0.01 picomoles per 100 ml. The radioactive probe ¹⁵I-tyrosyl-Aα 1-21 was diluted in assay buffer to yield approximately 10,000 cpm per 100 ml. The assay was set up in a total volume of 300 ml, with the volume made up where necessary with assay buffer. The assay included antibody-free controls to determine non-specific binding and controls containing antibody plus probe, without added specificity probe to determine the control level of probe binding. All determinations were made in duplicate. The assay samples and controls were processed and counted as described in Example 7. Percent control binding was determined by calculating the average cpm for the duplicate samples at each concentration of specificity probe, subtracting the average non-specific binding cpm, and dividing the results by the antibody-only counts. The results are shown in the following table.

**TABLE 7**

| Sensitivity of Antibody | | |
|---|---|---|
| Sample | ¹⁵I-CPM | Percent Control Binding |
| Amount of Unlabelled Probe (pmole) | | |
| 1.0 | 402 | |
| | 416 | 10.5 |
| 0.1 | 1,383 | |
| | 1,393 | 57.1 |
| 0.01 | 2,243 | |
| | 2,158 | 95.8 |
| Antibody only | 2,186 | |
| | 2,390 | |
| Non-specific | 193 | |
| | 185 | --- |
| Total cpm | 9,564 | |
| | 9,498 | --- |

The sensitivity assay shows that the amount of Aα 1-21 peptide capable of inhibiting the binding of radiolabeled probe by 50 percent is approximately 0.1 picomole.

### EXAMPLE 9

### Determination Of Antibody Specificity

Antibody specificity was determined by evaluating the ability of unlabelled peptides, specificity probes, of varying length, see Example 6, to inhibit the binding of the Aα 1-21radioactive probe. Rabbit antiserum prepared against the Aα 17-21 immunogen described in Example 2 was diluted in assay buffer, see Example 6, at a dilution of 1:1,000. The specificity peptides of Example 5 were diluted in assay buffer at concentrations ranging from 1.6 x 10⁻¹⁰ M to 1 x 10⁻⁵ M. One hundred ml of each dilution was added to 100 ml of antiserum and 100 ml of radioactive probe. This represents specificity probe peptide in concentrations ranging from 0.016 picomoles to 1.0 nanomoles in the 100 ml aliquot used in the assay. The radioactive probe ¹⁵I-tyrosyl-Aα 1-21, see Example 5, was diluted in assay buffer to yield approximately 20,000 cpm per 100 ml aliquot, the amount used per sample or control. The assay was set up in a volume of 300 ml, with the volume made up where necessary with assay buffer. The assay included antibody-free controls used to determine non-specific binding and controls containing antibody plus probe to determine the control level of probe binding. All determinations were made in duplicate. After overnight incubation at 4°C, non-antibody-bound radioactive probe was separated from antibody-bound radioactive probe by adsorption onto dextran-coated activated charcoal as described in Example 7. The assay included charcoal-free controls, to which 1 ml of PBS was added, for the determination of total counts.

The antibody-free control and the zero-peptide antibody control are counted to determine the 0% bound and the 100% bound values respectively. Radioactivity was determined using a gamma counter and standard techniques known in the art. The samples containing the test peptides are then counted and the amount of radioactivity associated with the antibody-free control subtracted from each. The resulting net counts are divided by the net counts in the antibody-only control to determine the percent bound in the presence of each amount of peptide. The following table shows the amount of each specificity probe necessary to inhibit antibody binding of labeled probe by 50 percent.

**TABLE 8**

| Antibody Specificity | | |
|---|---|---|
| Specificity Probe | Inhibitory Concentration (IC₅₀) picomoles | |
| | Rabbit (#20) | Guinea Pig (#3) |
| Aα 1-21 | 0.1 | 0.1 |
| Aα 2-21 | 0.1 | - |
| Aα 9-21 | 0.1 | - |
| Aα 12-21 | 0.1 | - |
| Aα 14-21 | 0.2 | - |
| Aα 15-21 | 0.2 | - |
| Aα 17-21 | 1.6 | 10 |
| Aα 17-21-NH₂ | > 1000.0 | 5000 |
| Aα 1-22 | > 1000.0 | - |
| Aα 1-20 | 600.0 | - |
| Aα 1-19 | 800.0 | - |
| Aα 1-18 | > 1000.0 | - |

The table lists the amount of each peptide required to inhibit radioactive probe binding by 50 percent, the inhibitory concentration. Only those specificity peptides which have the entire Aα 17-21 carboxyl sequence cross-react with the labeled probe Aα 1-21. It should be noted that the amidated analog of Aα 17-21 is not cross-reactive.

### EXAMPLE 10

### Immunoassay Protocol For The Determination Of Aα 17-21 Epitope Containing Peptide Concentration In Unknown Samples

The radioimmunoassay was conducted in a total volume of 300 ml with all dilutions carried out in assay buffer, Example 6. Standard solutions were prepared at concentrations in the range of 1 x 10⁻¹⁰M to 2 x 1O⁻⁸M which represents amounts of peptide ranging from 0.01 picomoles to 2.0 picomoles in the 100 ml aliquot used in the assay. The assay included antibody-free controls to measure non-specific binding, and controls containing antibody and probe without added standard samples or unknown samples to determine the control level of peptide binding. To 100 ml of buffer, standard sample or unknown was added, 100 ml of specific antiserum from Examples 2 and 7, diluted 1:1,000 in assay buffer. This was followed by the addition of 100 ml of radioactive probe, 125I-Tyrosyl-Aα 1-21 fibrinogen peptide, diluted in assay buffer to yield approximately 15,000 to 20,000 cpm per 100 ml aliquot. The assay was incubated overnight at 4°C and the non-antibody-bound radioactive probe was separated from antibody-bound probe by adsorption onto dextran-coated activated charcoal, see Example 7. Each assay included charcoal-free controls, to which 1 ml PBS was added for the determination of total counts. Radioactivity was determined using a gamma counter and standard techniques known in the art.

The antibody-free control and the zero-peptide antibody control were counted to determine the 0% bound and 100% bound values respectively. The assay standards are then counted and divided by the antibody control to determine the percent bound and a standard curve was generated. When the percent bound is plotted as a function of the logarithem of the amount of peptide in the standard, a sigmoidal curve is generated which is close to linear between the limits of 80% bound and 20% bound. Unknowns are counted, their percent of control calculated and they are compared to the standard curve to determine the amount of peptide present in the sample. Only those unknowns with values between 80% and 20% of control binding are considered valid.

### In Vitro Production of Cleavage Epitope Containing Peptide Aα17-21 In Human Blood. Effect of Ionophore Concentrations

Duplicate 1.0 ml aliquots of freshly-drawn heparinized vaccutainers containing 50 u per ml whole human blood were incubated for 15 minutes at 37°C in the presence of calcium ionophore A23187 at concentrations ranging up to 200 mM. The calcium ionophore was dissolved in dimethyl sulfoxide (DMSO) with the DMSO concentration in blood kept constant at 0.2%. Following incubation the aliquots were placed on ice, centrifuged and the plasma collected. Plasma aliquots (400 ml) were removed and placed in 12 x 75 mm glass tubes and 1200 ml chilled ethanol was added to precipitate the protein. The sample was centrifuged and 800 ml of the supernatant fluid was removed and evaporated to dryness in a Speed-vac concentrator. The sample was reconstituted to original plasma volume with 200 ml of distilled water. Duplicate 100 ml aliquots were assayed for Aα 17-21 epitope containing peptide (Aα 1-21) as described in this Example. The following table shows the amount of Aα 17-21 epitope containing peptide produced in response to by various amounts of ionophore.

**TABLE 9**

| Ionophore Stimulated Production of Aa 17-21 Epitope Containing Peptide From Whole Human Blood | |
|---|---|
| Ionophore Concentration (mM) | Aα 17-21 Epitope Containing (picomoles per ml blood) |
| 0 | 0 |
| 5 | 1.2 |
| 15 | 1.6 |
| 25 | 2.6 |
| 50 | 2.8 |
| 100 | 4.0 |
| 150 | 4.1 |
| 200 | 3.8 |

The optimal production of Aα 17-21 epitope-containing peptide occured following stimulation with 150 mM of calcium ionophore A23187.

### Ionophore Incubation Time

Calcium ionophore A23187 was added to replicate 1.0 ml aliquots of freshly-drawn heparinized whole blood as described above, to a final concentration of 150 mM. Additional control aliquots of blood received DMSO at a concentration of 0.2%. The blood aliquots were incubated at 37° for various time intervals ranging up to 60 minutes. Following the incubation, plasma was prepared, processed and assayed as above. The results are shown in the following table.

**TABLE 10**

| Time dependent Production of Aα 17-21 Epitope Containing Peptide From Whole Blood By Ionophore A23187 | |
|---|---|
| Incubation Time minutes | Aα 17-21 Epitope (picomoles/ml plasma) |
| <1.0 | 0.7 |
| 2.5 | 2.3 |
| 7.5 | 2.8 |
| 10.0 | 3.5 |
| 15.0 | 3.7 |
| 20.0 | 3.7 |
| 25.0 | 3.8 |
| 30.0 | 3.8 |
| 40.0 | 3.6 |
| 50.0 | 3.1 |
| 60.0 | 3.2 |

The concentration of Aα 17-21 epitope containing peptide increases with time and reaches a maximum concentration of between 3.5 and 4.0 mM between 10 and 40 minutes. Controls containing DMSO alone caused no peptide production. An incubation time of 25 minutes was selected for future experiments.

### Other Membrane Perturbators

Other known membrane perturbators have been used to evaluate the in vitro production of the epitope containing cleavage peptide Aα 17-21 in human blood. Aliquots of freshly-drawn heparinized whole blood were incubated for 25 minutes at 37°C in the presence of either 5 mg/ml cytochalasin B and concentrations of phorbol-12-myristate-13-acetate (PMA) ranging up to 20 mM or in the presence of the combination of 5 mg/ml cytochalasin B and concentrations of n-formyl-L-norleucyl-L-leucyl-L-phenylalanine (FNLP) ranging up to 20 mM. Plasma was collected, prepared and assayed as described above. The results are shown in the following tables.

**TABLE 11**

| PMA And Cytochalasin B Stimulated Production of Aα 17-21 Epitope Containing In Whole Human Blood | |
|---|---|
| PMA mMolar | Aα 17-21 Epitope Containing Peptide Produced (picomoles/ml plasma) |
| 0.003 | 0.0 |
| 0.010 | 0.3 |
| 0.03 | 0.3 |
| 0.10 | 0.4 |
| 0.30 | 0.9 |
| 1.0 | 1.5 |
| 3.0 | 2.6 |
| 10.0 | 3.0 |
| 20.0 | 3.7 |

**TABLE 12**

| FNLP And Cytochalasin B Stimulated Release of Aα 17-21 Epitope Containing Peptide From Whole Human Blood | |
|---|---|
| FNLP mMolar | Aα 17-21 Epitope Containing Peptide Produced (picomoles/ml plasma) |
| 0.001 | 0.0 |
| 0.003 | 0.0 |
| 0.010 | 0.0 |
| 0.03 | 0.4 |
| 0.1 | 0.9 |
| 0.3 | 1.1 |
| 1.0 | 1.1 |
| 3.0 | 1.3 |
| 10.0 | 1.2 |
| 20.0 | 1.3 |

### EXAMPLE 11

### Applications Of Aα 17-21 Epitope-Containing Peptide Radioimmunoassay

The following descriptions illustrate that the fibrinogen peptide immunoassay can be used to monitor the effective activity of human leukocyte elastase in human blood.

### Calcium Ionophore A23187-Stimulated Production of Fibrinogen Cleavage Peptide in The Blood of Normal Humans

Heparinized venous blood was drawn from seventeen normal healthy volunteers on three occasions over a five week period. On each occasion, calcium ionophore A23187 was added to replicate aliquots of the freshly drawn blood to a final concentration of 150 mM. The blood was incubated at 37° for 25 minutes, the plasma collected and assayed for cleavage peptide. A representative example of the results are shown in the following table.

**TABLE 13**

| Calcium Ionophore A23187 Stimulation Of Fibrinogen Cleavage Product In Normal Human Blood | | | |
|---|---|---|---|
| Picomoles Aα 17-21 | | | |
| Donor | 10⁶Neutrophils | | |
| | Week 1 | Week 3 | Week 5 |
| A.B. | 0.78 | 0.74 | 0.72 |
| K.B. | 0.81 | 1.07 | 2.04 |
| E.D. | 0.98 | 1.04 | 1.22 |
| P.D. | 0.69 | 0.90 | 1.25 |
| J.D. | 0.58 | 0.65 | 0.59 |
| C.D. | -- | 1.16 | 1.04 |
| P.F. | -- | 0.88 | 1.21 |
| D.F. | -- | 0.62 | 1.10 |
| W.H. | -- | 1.12 | 1.07 |
| J.H | 0.97 | 1.55 | 0.98 |
| A.K. | -- | 0.84 | -- |
| B.M. | -- | 0.55 | -- |
| J.N. | 1.40 | 1.23 | 1.00 |
| B.R. | 1.25 | 0.89 | -- |
| S.S. | 1.05 | 1.15 | 1.54 |
| K.T. | -- | 1.00 | 1.27 |

Normal individuals produced consistent amounts of fibrinogen cleavage peptide as determined by this assay. The dashed lines indicate that no sample was obtained from the individual at that time.

### Effect of An Elastase Inhibitor On Calcium Ionophore A23187-Stimulated Production of Fibrinogen Cleavage Peptide In The Blood Of Normal Humans

The effect of the elastase inhibitor 3-Acetoxymethyl-1a-methoxy-6-oxo-5-thia-1-azabicyclo[4.2.0]oct-2-ene-2-(2-(S)-carboxypyrrolidinecarboxamide)5,5-dioxide (compound 1) on the calcium ionophore A23187-induced fibrinogen cleavage peptide production was evaluated. Replicate 2 ml aliquots of freshly-drawn heparinized whole human blood were prepared with concentrations of compound 1 ranging up to 100 ml/ml. Following a brief pre-incubation of 5 minutes at 37°C, calcium ionophore A23187 was added to a final concentration of 150 mM. Non-ionophore containing blood and ionophore-only (no inhibitor) controls were included to measure the extent of uninhibited peptide generation. All aliquots were incubated at 37°C for 25 minutes, the plasma collected, processed and assayed for fibrinogen cleavage peptide as described above. The results are shown in the following table.

**TABLE 14**

| Elastase Inhibitor Activity Assay | | |
|---|---|---|
| Elastase Inhibitor mg/ml | Cleavage Product Produced picomoles/ml | Percent Inhibition |
| 0 | 2.4 | 0 |
| 1 | 2.3 | 4 |
| 5 | 1.6 | 33 |
| 10 | 1.6 | 33 |
| 20 | 1.5 | 37 |
| 30 | 1.2 | 50 |
| 40 | 0.8 | 67 |
| 80 | 0.4 | 85 |

The results demonstrate that a known elastase inhibitor is capable of inhibiting the ionophore-induced formation of the elastase cleavage product in human blood. This is consistant with the observation that exposure of whole human blood to calcium ionophore A23187 results in elastase cleavage product by allowing the release of neutrophil elastase.

### Effect of An Elastase Inhibitor On Calcium Ionophore A23187-Stimulated Production of Fibrinogen Cleavage Peptide In Primate Blood

Two chimpanzees were anesthetized and fitted with venous catheters for the collection of blood. Blood was collected from each animal at intervals over a 60 minute period prior to the administration of the elastase inhibitor. All blood samples were collected in heparin. After the initial 60 minute period one animal was given an intravenous injection of 10 mg/kg of the elastase inhibitor compound 1 while the other animal received an equivalent volume of normal saline. Additional heparinized blood samples were taken from both animals at regular intervals over the next 60 minutes. Each freshly-drawn sample of whole heparinized chimpanzee blood was divided into 4 aliquots. Calcium ionophore A23187 was added at a final concentration of 150 mM to two of the aliquots and an equivalent volume of the DMSO vehicle was added to the other two. All blood samples were incubated at 37° for 25 minutes, plasma collected and assayed as described above. The results are shown in the following table.

**TABLE 15**

| Ionophore-Induced Production of Aα 17-21 Epitope Containing Peptide in Chimpanzee Blood From An Elastase Inhibitor-Treated And Control Animal | | |
|---|---|---|
| | A 1-21 Peptide Produced (picomoles/ml plasma) | |
| Time (min) | Treated | Control |
| -60.0 | 2.70 | 0.80 |
| -45.0 | 3.50 | 1.00 |
| -30.0 | 3.10 | 0.81 |
| -15.0 | 3.00 | 0.95 |
| 0.0 | 3.00 | 0.98 |
| 1.0 | 1.10 | 1.20 |
| 2.5 | 1.30 | 1.10 |
| 5.0 | 1.60 | 0.97 |
| 7.5 | 2.00 | 1.10 |
| 10.0 | 1.80 | 1.20 |
| 12.5 | 1.70 | 1.10 |
| 15.0 | 0.98 | 1.50 |
| 20.0 | 1.90 | 1.50 |
| 30.0 | 2.20 | 1.20 |
| 40.0 | 3.10 | 1.50 |
| 50.0 | 2.60 | 1.20 |
| 60.0 | 2.50 | 0.96 |

Elastase inhibitor was infused into the treated animal at time 0.

The blood samples drawn from the treated chimpanzee after infusion of the elastase inhibitor produced markedly lower levels of the fibrinogen peptide in response to calcium ionophore A23187. Fibrinogen cleavage peptide was not detected in the non-ionophore treated blood samples from either animal. Over the course of 30 to 40 minutes, the amount of ionophore-stimulated peptide production in freshly-drawn samples gradually returned to the pretreatment level. No consistent change over time was observed in the untreated animal.

### Calcium Ionophore A23187-Stimulated Production of Fibrinogen Cleavage Peptide in The Blood Of Alpha-1 Proteinase Deficient Humans

Heparinized venous blood was drawn from four individuals who process the PiZZ phenotype, deficient for alpha-1 proteinase inhibitor. Blood was also collected from two normal volunteers. Calcium ionophore A23187 was added to replicate aliquots of the freshly drawn blood to a final concentration of 150 mM. The blood was incubated at 37° for 25 minutes, the plasma collected and assayed cleavage peptide. The results are shown in the following table.

**TABLE 16**

| Production of Aα 17-21 Epitope Containing Peptide In Whole Blood of PiZZ and Normal Individuals In the Presence of Calcium Ionophore | | |
|---|---|---|
| Patient | Control | A23187 |
| Normal | <0.4 | 4.2 |
| Normal | <0.5 | 4.6 |
| PiZZ | <0.7 | 8.4 |
| PiZZ | <0.6 | 26.5 |
| PiZZ | <0.5 | 20.5 |
| PiZZ | <0.4 | 17.8 |

Values are expressed as pM Aα 17-21 epitope containing peptide produced per ml of plasma. The blood samples derived from the four individuals who were genetically deficient in alpha-1 proteinase inhibitor produced greater amounts of peptide than did those derived from the normal volunteers. Under basal conditions no fibrinogen cleavage products could be detected in the blood of any of the donors.

## Claims

1. An isolated and purified peptide comprising the carboxyl terminus of the primary cleavage products of human leukocyte elastase cleaved human fibrinogen which is capable of inducing specific antibodies against said cleavage products and acting as a specific probe for the detection of said antibodies, said peptide antigen having one of the following epitopes at its carboxyl terminus: 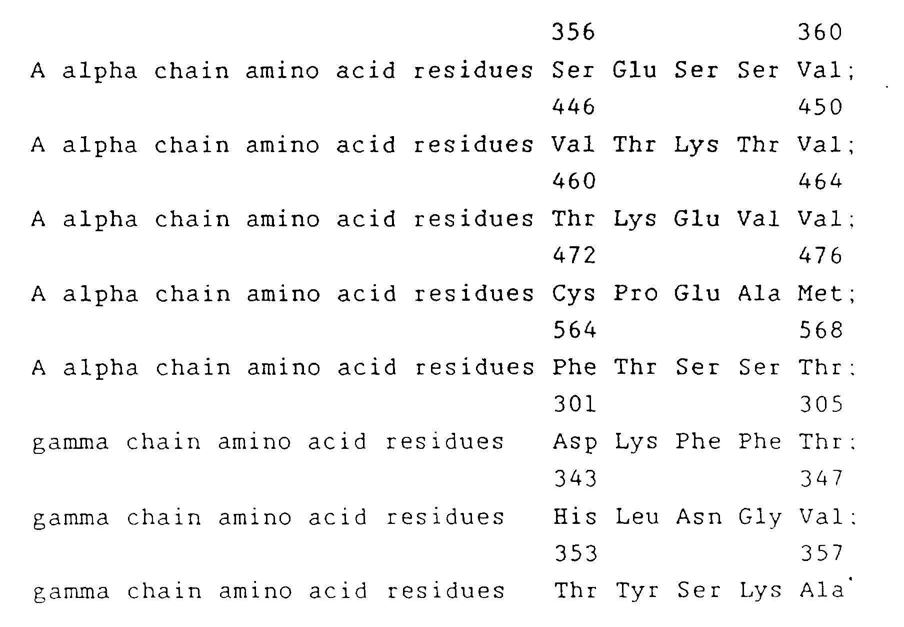 or acid addition salts thereof.

2. An isolated and purified peptide comprising the amino terminus of the primary cleavage products of human leukocyte elastase cleaved human fibrinogen which is capable of inducing specific antibodies against said cleavage products and acting as a specific probe for the detection of said antibodies, said peptide antigen having one of the following epitopes at its amino terminus: 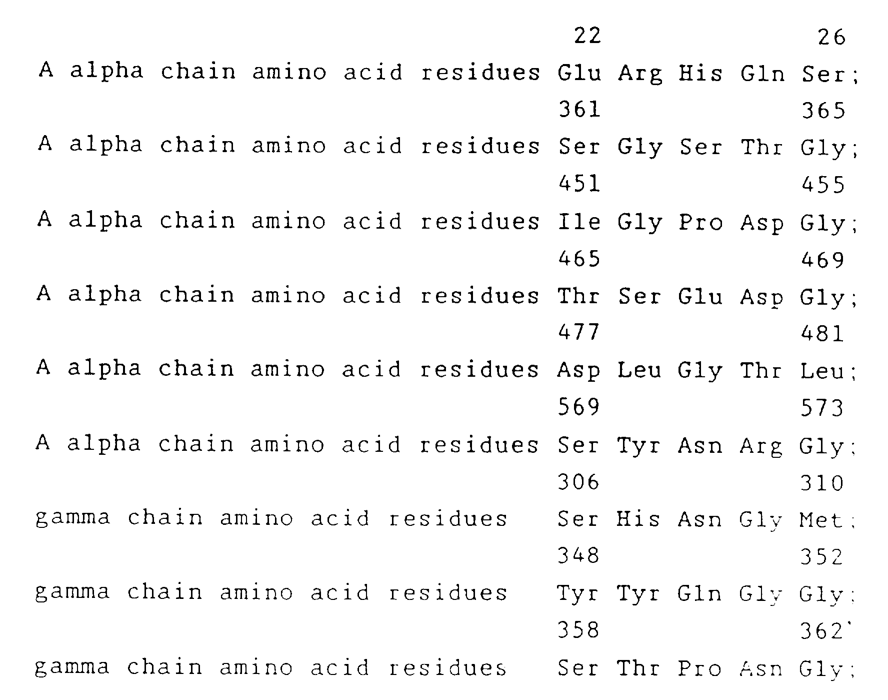 or the amide or acid addition salts thereof.

3. A peptide immunogen comprising a peptide of Claim 1 coupled to a linking amino acid if necessary and a marker amino acid, with said linking and marker amino acids attached to the amino terminal amino acid residue of the peptide epitope, with either the linking amino acid or the marker amino acid being directly attached to the amino terminal amino acid residue and the linking amino acid attached to a carrier protein.

4. A peptide immunogen comprising a peptide of Claim 2 coupled to a linking amino acid if necessary and a marker amino acid, with said linking and marker amino acids attached to the carboxyl terminal amino acid residue of the peptide epitope, with either the linking amino acid or the marker amino acid being directly attached to the amino terminal amino acid residue and the linking amino acid attached to a carrier protein.

5. The peptide of Claim 1
wherein the specificity is determined by the amino acid sequence of the carboxyl terminus of the epitope.

6. The peptide of Claim 2
wherein the specificity is determined by the amino acid sequence of the amino terminues of the epitope.

7. The linking amino acid of Claim 3 wherein the amino acid is cysteine.

8. The marker amino acid of Claim 4 wherein the amino acid is norleucine.

9. Monospecific antibody raised against the peptide epitopes of Claim 1 or Claim 2.

10. The monospecific antibody of Claim 9 wherein the antibody is obtained form the blood of animals immunized with specific immunogen.

11. The probe peptide of Claim 1 wherein there is attached to the amino terminal amino acid a linker amino acid which will allow the attachment of a marker substance.

12. The linker amino acid of Claim 11 wherein the amino acid is tyrosine.

13. A method for detecting the presence of leukocyte elastase induced fibrinogen cleavage epitope-containing peptides in whole blood samples or leukocyte containing body fluid samples, comprising the following steps:
a) combining the sample with an anticoagulant; then
b) incubating the sample with a leukocyte membrane perturbating agent for a period sufficient for the release of elastase from the leukocytes and to cleave the fibrinogen, then
c) isolating the fibronogen cleavage product eptiope-containing peptides by centrifugation and collection of the plasma, then
d) contacting the epitope-containing plasma sample with at least one monospecific antibody specifically reactive with an elastase-induced fibrinogen cleavage product epitope to form an epitope-antibody mixture, then
e) measuring the extent of the reaction by serological or immunological assays and determining a value; then
f) comparing the values of experimental samples with standard values.

14. A method for detecting the presence of leukocyte elastase induced fibrinogen cleavage epitope-containing peptides in whole blood samples or leukocyte containing body fluid samples comprising the following steps:
a) combining the blood sample with an anticoagulant; then
b) incubating of the blood sample with a leukocyte membrane perturbating agent for a period sufficient for the release of elastase from the elukocytes and to cleave the fibrinogen; then
c) isolating the fibrinogen cleavage product epitope containing peptides by centrifugation and collection of the plasma; then
d) precipitation of the plasma proteins by treating with alcohol, centrifugation and evaporation of the supernatant fluid to dryness; then
e) reconstitution of the plasma supernatant fluid, then
f) contacting the reconstituted plasma proteins with at least one monospecific antibody specifically reactive with an elastase induced fibrinogen cleavage product epitope to form an epitope-antibody mixture; then
g) measuring the extent of the reaction by serological or immunological assays and determining a value; and
h) comparing the values of experimental samples with standard values.

15. A method for detecting the presence of leukocyte elastase induced fibrinogen cleavage product epitopes in body fluids which contain fibrinogen but do not contain leukocytes, comprising the following steps:
a) addition of leukocytes or leukocyte elastase to the sample;
b) when leukocytes are added the sample is incubated with a leukocyte membrane perturbating agent for a period sufficient for the release of elastase from the leukocytes and to cleave the fibrinogen;
c) isolation of the fibrinogen cleavage product eptiope containing peptides from the sample;
d) contacting the elastase-containing plasma sample with at least one monospecific antibody specifically reactive with an elastase-induced fibrinogen cleavage product epitope to form an epitope-antibody mixture; then
e) measuring the extent of the reaction by serological or immunological assays and determining a value; and
f) comparing the values of experimental samples with standard values.

16. The method of any oneof Claims 13-15 step b, wherein said membrane perturbating agent is selected from the group consisting of: zymosan; antigen-antibody complexes, complement C5a; N-formyl-L-methionyl-L-leucyl-L-phenylalanine plus cytochalasin B;phorbol-12-myristate-13-acetate plus cytochalasin B; n-formyl-L-norleucyl-L-leucyl-L-phenylalanine plus cytochalasin B or calcium ionophore A23187.

17. The method of Claim 16 wherein the membrane perturbating agent is calcium ionophore A23187.

18. A method of any one of Claims 13-15, step d, wherein the antibody is reactive with the specific epitope of Claim 1 or Claim 2.

19. The method of any one of Claims 13-15, step e, wherein the assay includes the following steps:
a) contacting the epitope-antibody mixture with a labeled probe complementary to the specific antibody; then
b) incubation of the epitope-antibodyprobe mixture; and
c) separation of the antibody-bound probe from the non-antibody-bound probe and determination of the value of antibody-bound probe.

20. The method of Claim 19, step b, wherein the incubation is at about 4°C for about 10 to about 20 hours or at about 37° for about 10 minutes to about 2 hours.

21. A method of Claim 19, step c, wherein the method of separating the non-antibody-bound probe is by chromatographic, precipitation, immunoprecipitation or adsorption techniques.

22. A method of Claim 21 wherein the method of separating the non-antibody-bound probe is by adsorption onto dextran-coated activated charcoal.

23. The probe peptide of Claim 4 wherein there is attached to the carboxyl terminal amino acid a linker amino acid which will allow the attachment of a marker substance.

24. The linker amino acid of Claim 23 wherein the amino acid is tyrosine.

## Patentansprüche

1. Ein isoliertes und gereinigtes Peptid, umfassend den Carboxyterminus der primären Spaltprodukte von mit menschlicher Leukozytenelastase gespaltenem menschlichern Fibrinogen, das spezifische Antikörper gegen die Spaltprodukte induzieren und als eine spezifische Sonde für den Nachweis dieser Antikörper wirken kann, wobei das Peptidantigen eines der folgenden Epitope an seinem Carboxyterminus besitzt: 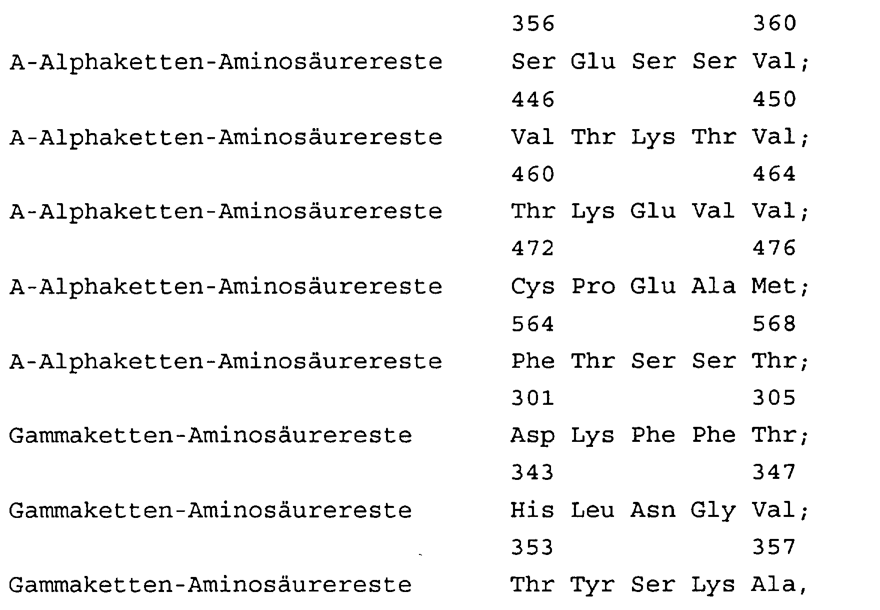 oder Säureadditionssalze davon.

2. Ein isoliertes und gereinigtes Peptid, umfassend den Aminoterminus der primären Spaltprodukte von mit menschlicher Leukozytenelastase gespaltenem menschlichem Fibrinogen, das spezifische Antikörper gegen diese Spaltprodukte induzieren und als eine spezifische Sonde für den Nachweis dieser Antikörper wirken kann, wobei das Peptidantigen eines der folgenden Epitope an seinem Aminoterminus besitzt: 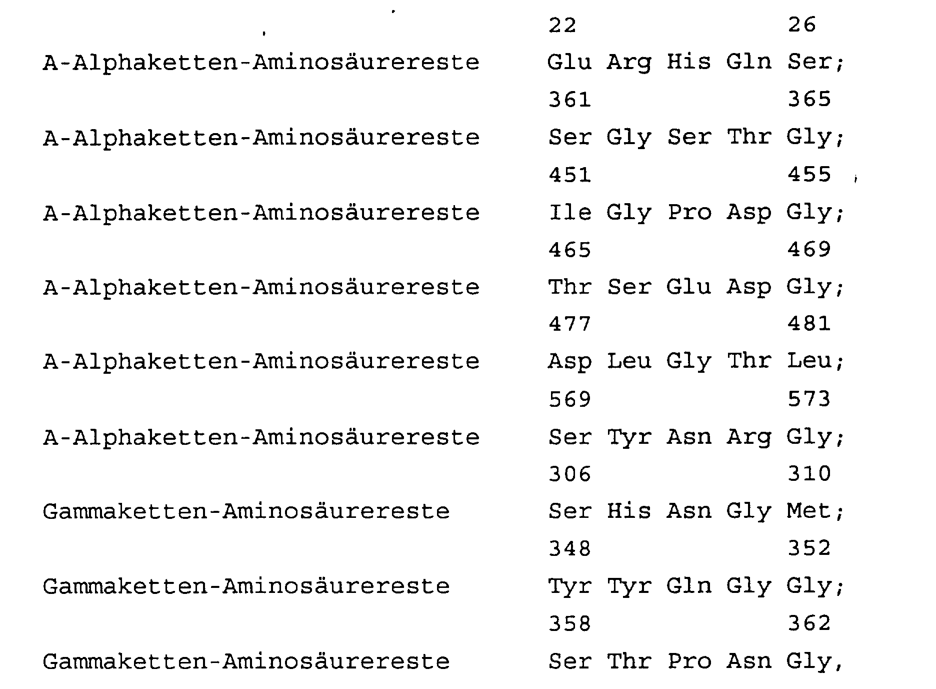 oder die Amid- oder Säureadditionssalze davon.

3. Ein Peptidimmunogen, umfassend ein Peptid nach Anspruch 1, das an eine verbindungsaminosäure falls notwendig und eine Markierungsaminosäure gekuppelt ist, wobei die Verbindungs- und Markierungsaminosäure an den aminoterminalen Aminosäurerest des Peptidepitopes gebunden sind und wobei entweder die verbindungsaminosäure oder die Markierungsaminosäure direkt an den aminoterminalen Aminosäurerest gebunden ist und die Verbindungsaminosäure an ein Trägerprotein gebunden ist.

4. Ein Peptidimmunogen, umfassend ein Peptid nach Anspruch 2, das an eine Verbindungsaminosäure falls notwendig und eine Markierungsaminosäure gekuppelt ist, wobei die Verbindungs- und Markierungsaminosäure an den carboxyterminalen Aminosäurerest des Peptidepitops gebunden sind und wobei entweder die Verbindungsaminosäure oder die Markierungsaminosäure direkt an den aminoterminalen Aminosäure rest gebunden ist und die Verbindungsaminosäure an ein Trägerprotein gebunden ist.

5. Das Peptid nach Anspruch 1, wobei die Spezifität durch die Aminosäuresequenz des Carboxyterminus des Epitops bestimmt wird.

6. Das Peptid nach Anspruch 2, wobei die Spezifität durch die Aminosäuresequenz des Aminoterminus des Epitops bestimmt wird.

7. Die Verbindungsaminosäure nach Anspruch 3, wobei die Aminosäure Cystein ist.

8. Die Markierungsaminosäure nach Anspruch 4, wobei die Aminosäure Norleucin ist.

9. Monospezifischer Antikörper, der gegen die Peptidepitope nach Anspruch 1 oder Anspruch 2 gebildet ist.

10. Der monospezifische Antikörper nach Anspruch 9, wobei der Antikörper aus dem Blut von Tieren erhalten wird, die mit spezifischem Immunogen immunisiert sind.

11. Das Sondenpeptid nach Anspruch 1, wobei an die aminoterminale Aminosäure eine Verbindungsaminosäure gebunden ist, die die Bindung einer Markierungsubstanz ermöglicht.

12. Die Verbindungsaminosäure nach Anspruch 11, wobei die Aminosäure Tyrosin ist.

13. Ein Verfahren zum Nachweis der Anwesenheit von Peptiden mit leukozytenelastaseinduziertem Fibrinogenspaltungsepitop in Gesamtblutproben oder leukozytenenthaltenden Körperflüssigkeitsproben, umfassend die folgenden Schritte:
a) Vereinigen der Probe mit einem Antikoagulans, anschließend
b) Inkubieren der Probe mit einem Mittel, das die Leukozytenmembran stört, für einen Zeitraum, der ausreicht, um Elastase aus den Leukozyten freizusetzen und das Fibrinogen zu spalten, anschließend
c) Isolieren der Peptide mit Fibrinogenspaltproduktepitop durch Zentrifugation und Sammeln des Plasmas, anschließend
d) Inkontaktbringen der epitopenthaltenden Plasmaprobe mit mindestens einem monospezifischen Antikörper, der spezifisch mit einem elastaseinduzierten Fibrinogenspaltproduktepitop reagiert unter Bildung eines Epitop-Antikörper-Gemischs, anschließend
e) Messen des Reaktionsgrades über serologische oder immunologische Tests und Bestimmen eines Wertes, anschließend
f) Vergleichen der Werte der Versuchsproben mit Standardwerten.

14. Ein Verfahren zum Nachweis der Anwesenheit von Peptiden mit leukozytenelastaseinduziertem Fibrinogenspaltungsepitop in Gesamtblutproben oder leukozytenenthaltenden Körperflüssigkeitsproben, umfassend die folgenden Schritte:
a) Vereinigen der Blutprobe mit einem Antikoagulans, anschließend
b) Inkubieren der Blutprobe mit einem Mittel, das die Leukozytenmembran stört, für einen Zeitraum, der ausreicht, um Elastase aus den Leukozyten freizusetzen und das Fibrinogen zu spalten, anschließend
c) Isolieren der Peptide mit Fibrinogenspaltproduktepitop durch Zentrifugation und Sammeln des Plasmas, anschließend
d) Fällung der Plasmaproteine durch Behandlung mit Alkohol, Zentrifugation und Verdampfen der Überstandsflüssigkeit bis zur Trockne, anschließend
e) Wiederherstellen der Plasma-Überstandsflüssigkeit, anschließend
f) Inkontaktbringen des wiederhergestellten Plasmaproteins mit mindestens einem monospezifischen Antikörper, der spezifisch mit einem elastaseinduzierten Fibrinogenspaltproduktepitop reagiert unter Bildung eines Epitop-Antikörper-Gemischs, anschließend
g) Messen des Reaktionsgrades über serologische oder immunologische Tests und Bestimmen eines Wertes, und
h) Vergleichen der Werte der Versuchsproben mit Standardwerten.

15. Ein Verfahren zum Nachweis der Anwesenheit von leukozytenelastaseinduziertem Fibrinogenspaltproduktepitopen in Körperflüssigkeiten, die Fibrinogen enthalten, aber keine Leukozyten enthalten, umfassend die folgenden Schritte:
a) Zugabe von Leukozyten oder Leukozytenelastase zur Probe;
b) Wenn Leukozyten hinzugegeben werden, wird die Probe mit einem leukozytenmembranstörenden Mittel für einen Zeitraum inkubiert, der ausreicht, um Elastase aus den Leukozyten freizusetzen und das Fibrinogen zu spalten;
c) Isolierung der Fibrinogenspaltproduktepitop enthaltenden Peptide aus der Probe;
d) Inkontaktbringen der elastaseenthaltenden Plasmaprobe mit mindestens einem monospezifischen Antikörper, der spezifisch mit einem elastaseinduzierten Fibrinogenspaltproduktepitop reagiert unter Bildung eines Epitop-Antikörper-Gemischs; anschließend
e) Messen des Reaktionsgrades über serologische oder immunologische Tests und Bestimmen eines Wertes, und
f) vergleichen der Werte der Versuchsproben mit Standardwerten.

16. Das Verfahren nach einem der Ansprüche 13-15, Schritt b, wobei das membranstörende Mittel aus der Gruppe ausgewählt wird, bestehend aus: Zymosan; Antigen-Antikörper-Komplexen, Komplement C5a; N-Formyl-L-methionyl-L-leucyl-L-phenylalanin plus Cytochalasin B; Phorbol-12-myristat-13-acetat plus Cytochalasin B; N-Formyl-L-norleucyl-L-leucyl-L-phenylalanin plus Cytochalasin B oder Calciumionophor A23187.

17. Das Verfahren nach Anspruch 16, wobei das membranstörende Mittel Calciumionophor A23187 ist.

18. Ein Verfahren nach einem der Ansprüche 13-15, Schritt d, wobei der Antikörper mit dem spezifischen Epitop nach Anspruch 1 oder Anspruch 2 reagiert.

19. Das Verfahren nach einem der Ansprüche 13-15, Schritt e, wobei die Untersuchung die folgenden Schritte umfaßt:
a) Inkontaktbringen des Epitop-Antikörper-Gemischs mit einer markierten Sonde, die komplementär zu dem spezifischen Antikörper ist; anschließend
b) Inkubation des Epitop-Antikörper-Sonden-Gemischs; und
c) Trennung der antikörpergebundenen Sonde von der nichtantikörpergebundenen Sonde und Bestimmung der Menge der antikörpergebundenen Sonde.

20. Das Verfahren nach Anspruch 19, Schritt b, wobei die Inkubation bei ca. 4°C ca. 10 bis ca. 20 Stunden lang dauert oder bei ca. 37°C ca. 10 Minuten bis ca. 2 Stunden dauert.

21. Ein Verfahren nach Anspruch 19, Schritt c, wobei das Trennverfahren der nichtantikörpergebundenen Sonde über chromatographische, Fällungs-, Immunofällungs- oder Adsorptionstechniken erfolgt.

22. Ein Verfahren nach Anspruch 21, wobei das Trennverfahren der nichtantikörpergebundenen Sonde über Adsorption an dextranbeschichteter Aktivkohle erfolgt.

23. Das Sondenpeptid nach Anspruch 4, wobei an die carboxyterminale Aminosäure eine Verbindungsaminosäure gebunden ist, die die Bindung einer Markierungssubstanz ermöglicht.

24. Die Verbindungsaminosäure nach Anspruch 23, wobei die Aminosäure Tyrosin ist.

## Revendications

1. Peptide isolé et purifié comportant l'extrémité carboxylique des produits issus du clivage primaire du fibrinogène humain clivé par l'élastase des leucocytes humains qui est capable d'induire des anticorps spécifiques dirigés contre lesdits produits issus du clivage et d'agir comme une sonde spécifique pour la détection desdits anticorps, ledit antigène peptidique ayant un des épitopes suivants au niveau de son extrémité carboxylique : 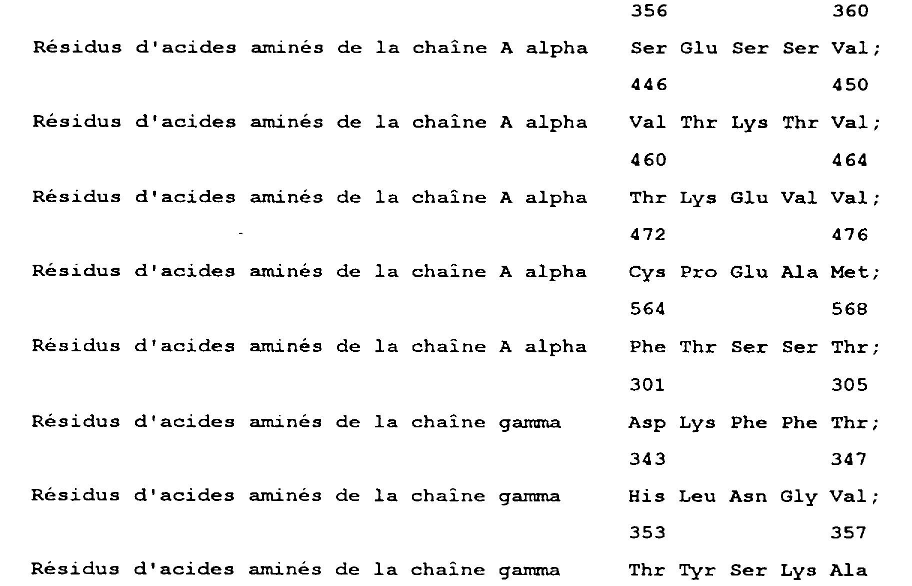 ou des sels acides d'addition de ceux-ci.

2. Peptide isolé et purifié comportant l'extrémité amine des produits issus du clivage primaire du fibrinogène humain clivé par l'élastase des leucocytes humains qui est capable d'induire des anticorps spécifiques dirigés contre lesdits produits issus du clivage et d'agir comme une sonde spécifique pour la détection desdits anticorps, ledit antigène peptidique ayant un des épitopes suivants au niveau de son extrémité amine : 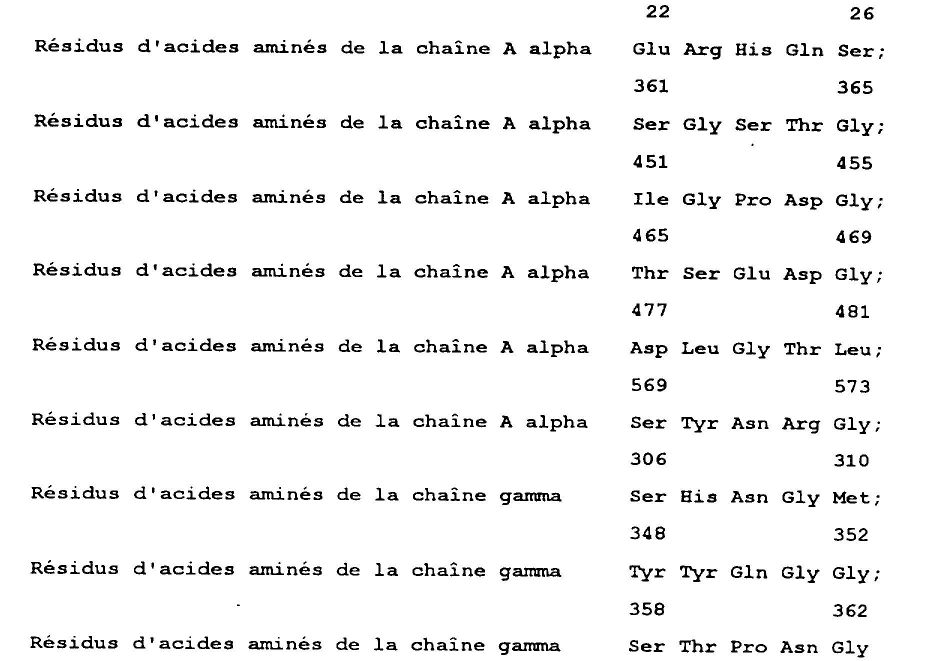 ou les amides ou les sels acides d'addition de ceux-ci.

3. Immunogène peptidique comportant un peptide selon la revendication 1, couplé à un acide aminé de liaison si nécessaire et à un acide aminé de type marqueur, lesdits acides aminés de liaison et de type marqueur étant attachés au résidu d'acide aminé de l'extrémité amine terminale de l'épitope peptidique, et l'acide aminé de liaison ou l'acide aminé de type marqueur étant directement attaché au résidu d'acide aminé de l'extrémité amine terminale et l'acide aminé de liaison étant attaché à une protéine de type support.

4. Immunogène peptidique comportant un peptide selon la revendication 2, couplé à un acide aminé de liaison si nécessaire et à un acide aminé de type marqueur, lesdits acides aminés de liaison et de type marqueur étant attachés au résidu d'acide aminé de l'extrémité carboxylique terminale de l'épitope peptidique, et l'acide aminé de liaison ou l'acide aminé de type marqueur étant directement attaché au résidu d'acide aminé de l'extrémité amine terminale et l'acide aminé de liaison étant attaché à une protéine de type support.

5. Peptide selon la revendication 1, dans lequel la spécificité est déterminée par la séquence en acides aminés de l'extrémité carboxylique de l'épitope.

6. Peptide selon la revendication 1, dans lequel la spécificité est déterminée par la séquence en acides aminés de l'extrémité amine de l'épitope.

7. Acide aminé de liaison selon la revendication 3, dans lequel l'acide aminé est la cystéine.

8. Acide aminé de type marqueur selon la revendication 4, dans lequel l'acide aminé est la norleucine.

9. Anticorps monospécifique dirigé contre les épitopes peptidiques selon la revendication 1 ou la revendication 2.

10. Anticorps monospécifique selon la revendication 9, dans lequel l'anticorps est obtenu à partir de sang d'animaux immunisés à l'aide d'un immunogène spécifique.

11. Peptide de sonde selon la revendication 1, dans lequel un acide aminé de liaison est attaché à l'acide aminé de l'extrémité amine, acide aminé de liaison qui permettra l'attachement d'une substance de type marqueur.

12. Acide aminé de liaison selon la revendication 11, dans lequel l'acide aminé est la tyrosine.

13. Procédé de détection de la présence d'un peptide contenant un épitope issu du clivage du fibrinogène induit par l'élastase des leucocytes dans des échantillons de sang total ou dans des échantillons de fluide corporel contenant des leucocytes, comportant les étapes suivantes consistant à :
a) combiner l'échantillon à l'anticoagulant; puis
b) mettre à incuber l'échantillon avec un agent de perturbation des membranes des leucocytes pendant une période de temps suffisante afin de libérer l'élastase des leucocytes et afin de cliver le fibrinogène, puis
c) isoler les peptides contenant un épitope d'un produit issus du clivage du fibrinogène par centrifugation et récolte du plasma, puis
d) mettre en contact l'échantillon de plasma contenant un épitope et au moins un anticorps monospécifique qui réagit de manière spécifique avec un épitope d'un produit issu du clivage du fibrinogène induit par l'élastase pour former un mélange épitope-anticorps, puis
e) mesurer l'étendue de la réaction par des essais sérologiques ou immunologiques et déterminer une valeur; puis
f) comparer les valeurs des échantillons expérimentaux à des valeurs standards.

14. Procédé de détection de la présence de peptide, contenant un épitope, issu du clivage du fibrinogène induit par l'élastase des leucocytes dans des échantillons de sang total ou dans des échantillons de fluide corporel contenant des leucocytes comportant les étapes suivantes consistant à :
a) combiner l'échantillon sanguin et un anticoagulant; puis
b) mettre à incuber l'échantillon sanguin avec un agent de perturbation des membranes des leucocytes pendant une période de temps suffisante afin de libérer l'élastase des leucocytes et afin de cliver le fibrinogène, puis
c) isoler les peptides contenant un épitope d'un produit issus du clivage du fibrinogène par centrifugation et récolte du plasma, puis
d) précipiter les protéines du plasma par un traitement avec de l'alcool, une centrifugation et une évaporation du fluide surnageant jusqu'à obtention d'un produit sec; puis
e) reconstituer le fluide surnageant du plasma, puis
f) mettre en contact les protéines du plasma reconstitué et au moins un anticorps monospécifique qui réagit de manière spécifique avec un épitope d'un produit issu du clivage du fibrinogène induit par l'élastase pour former un mélange épitope-anticorps; puis
g) mesurer l'étendue de la réaction par des essais sérologiques ou immunologiques et déterminer une valeur; et
h) comparer les valeurs des échantillons expérimentaux à des valeurs standards.

15. Procédé de détection de la présence d'épitopes d'un produit issu du clivage du fibrinogène induit par l'élastase des leucocytes dans des fluides corporels qui contiennent du fibrinogène mais qui ne contiennent pas de leucocytes, comportant les étapes suivantes consistant à :
a) ajouter des leucocytes ou une élastase de leucocytes à l'échantillon;
b) mettre à incuber l'échantillon, lorsque des leucocytes sont ajoutés, avec un agent de perturbation des membranes des leucocytes pendant une période de temps suffisante afin de libérer l'élastase à partir des leucocytes et afin de cliver le fibrinogène;
c) isoler les peptides contenant un épitope d'un produit issu du clivage du fibrinogène à partir de l'échantillon;
d) mettre en contact l'échantillon de plasma contenant l'élastase et au moins un anticorps monospécifique qui réagit de manière spécifique avec un épitope d'un produit issu du clivage du fibrinogène induit par l'élastase afin de former un mélange épitope-anticorps; puis
e) mesurer l'étendue de la réaction par des essais sérologiques ou immunologiques et déterminer une valeur; et
f) comparer les valeurs des échantillons expérimentaux à des valeurs standards.

16. Procédé selon l'une quelconque des revendications 13 à 15, étape b, dans lequel ledit agent de perturbation membranaire est sélectionné parmi le groupe constitué du zymosan; des complexes antigène-anticorps, du complément C5a; de la N-formyl-L-méthionyl-L-leucyl-L-phénylalanine plus de la cytochalasine B; du phorbol-12-myristate-13-acétate plus de la cytochalasine B; de la n-formyl-L-norleucyl-L-leucyl-L-phénylalanine plus de la cytochalasine B ou de l'ionophore de calcium A23187.

17. Procédé selon la revendication 16, dans lequel l'agent de perturbation membranaire est l'ionophore de calcium A23187.

18. Procédé selon l'une quelconque des revendications 13 à 15, étape d, dans lequel l'anticorps réagit avec l'épitope spécifique selon la revendication 1 ou la revendication 2.

19. Procédé selon l'une quelconque des revendications 13 à 15, étape e, dans lequel l'essai inclut les étapes suivantes consistant à :
a) mettre en contact le mélange épitope-anticorps et une sonde marquée complémentaire de l'anticorps spécifique; puis
b) mettre à incuber le mélange épitope-anticorps-sonde; et
c) séparer la sonde liée à l'anticorps de la sonde non-liée à l'anticorps et déterminer la valeur de la sonde liée à l'anticorps.

20. Procédé selon la revendication 19, étape b, dans lequel l'incubation se fait à environ 4°C pendant environ 10 à environ 20 heures ou à environ 37°c pendant environ 10 minutes à environ 2 heures.

21. Procédé selon la revendication 19, étape c, dans lequel le procédé de séparation de la sonde non-liée à l'anticorps est réalisé à l'aide de techniques chromatographiques, de techniques de précipitation, d'immunoprécipitation ou d'adsorption.

22. Procédé selon la revendication 21, dans lequel le procédé de séparation de la sonde non-liée à l'anticorps est réalisée par adsorption sur charbon activé revêtu de dextran.

23. Peptide de sonde selon la revendication 4, dans lequel un acide aminé de liaison est attaché à l'acide aminé de l'extrémité carboxylique, acide aminé de liaison qui permettra l'attachement d'une substance de type marqueur.

24. Acide aminé de liaison selon la revendication 23, dans lequel l'acide aminé est la tyrosine.
